# EUROPEAN PATENT SPECIFICATION

(11) **EP 2 115 124 B1**
(45) Date of publication and mention of the grant of the patent: **16.03.2016**
(21) Application number: 08705809.5
(22) Date of filing: 09.01.2008
(51) Int. Cl.: C12N 5/00

(54) **METHODS FOR INCREASING AND MOBILIZING HEMATOPOIETIC STEM CELLS**
VERFAHREN ZUR VERMEHRUNG UND MOBILISIERUNG BLUTBILDENDER STAMMZELLEN
PROCÉDÉS D'ACCROISSEMENT ET DE MOBILISATION DE CELLULES SOUCHES HÉMATOPOIETIQUES

(30) Priority: 09.01.2007 US 884162 P; 14.02.2007 US 889893 P; 17.05.2007 US 938564 P; 12.12.2007 US 13243
(43) Date of publication of application: 11.11.2009
(73) Proprietor: Cleveland Biolabs, Inc., Buffalo, NY 14203 (US)
(72) Inventor: SHAKHOV, Alexander, Buffalo, NY 14203 (US); STROM, Evguenia, Buffalo, NY 14203 (US)
(74) Representative: Grund, Martin
(86) International application number: PCT/US2008/050644
(87) International publication number: WO 2008/086426

(56) References cited:
- WO-A2-2006/138238
- US-A- 5 192 553
- BENNER R ET AL: "GENETIC CONTROL OF LIPO POLY SACCHARIDE INDUCED MOBILIZATION OF HEMOPOIETIC STEM CELLS DISSOCIATION BETWEEN EARLY AND DELAYED MOBILIZATION OF HEMOPOIETIC STEM CELLS IN COMPLEMENT C-5 DEFICIENT MICE AND LIPO POLY SACCHARIDE NONRESPONDER MICE", CELL AND TISSUE KINETICS, vol. 14, no. 2, 1981, pages 143-152, XP009158005, ISSN: 0008-8730
- NAGAI YOSHINORI ET AL: "Toll-like receptors on hematopoietic progenitor cells stimulate innate immune system replenishment", IMMUNITY, vol. 24, no. 6, June 2006 (2006-06), pages 801-812, XP002672552, ISSN: 1074-7613
- WRIGHT DOUGLAS E ET AL: "Physiological migration of hematopoietic stem and progenitor cells", SCIENCE (WASHINGTON D C), vol. 294, no. 5548, 30 November 2001 (2001-11-30), pages 1933-1936, XP002672551, ISSN: 0036-8075
- MASSBERG STEFFEN ET AL: "Immunosurveillance by hematopoietic progenitor cells trafficking through blood, lymph, and peripheral tissues", CELL, vol. 131, no. 5, 30 November 2007 (2007-11-30), pages 994-1008, XP002672553, ISSN: 0092-8674

## Description

### FIELD OF THE INVENTION

This invention relates to the use of an agent to increase the number of hematopoietic stem cells and mobilize these cells to the bloodstream.

### BACKGROUND OF THE INVENTION

Blood cells are responsible for constant maintenance and immune protection of every cell type of the body. Blood cells, along with skin cells, have the greatest powers of self-renewal of any adult tissue. The stem cells that form blood and immune cells are known as hematopoietic stem cells (HSC). They are ultimately responsible for the constant renewal of blood by producing billions of new blood cells each day. There two hallmarks of an HSC: it can renew itself and it can produce cells that give rise to all the different types of blood cells. Further research has shown that following reconstitution, HSC can differentiate not only into blood cells, but also muscle cells (both skeletal myocytes and cardiomyocytes), brain cells, liver cells, skin cells, lung cells, kidney cells, intestinal cells, and pancreatic cells.

Bone marrow is the classic source of HSC but it is infrequently used as a source due to an invasive harvesting procedure that requires general anesthesia for the stem cell donor. During the clinical procedure for bone marrow transplantation, a bone is punctured typically a hipbone and the bone marrow cells are drawn out with a syringe. About 1 in every 100,000 cells in the marrow is a long-term, blood-forming stem cell.

For clinical transplantation of human HSC, doctors now prefer to harvest donor cells from peripheral, circulating blood. It has been known for decades that a small number of stem and progenitor cells circulate in the bloodstream, but in the past 10 years, researchers have found that the cells can be coaxed to migrate from marrow to blood in greater numbers by injecting the donor with a cytokine, such as granulocyte-colony stimulating factor (G-CSF). The donor is injected with G-CSF a few days before the cell harvest. To collect the cells, an intravenous tube is inserted into the donor's vein and their blood is passed through a filtering system that pulls out CD34⁺ white blood cells and returns the red blood cells to the donor. This procedure is commonly referred as "stem cells aphaeresis". As is true for bone marrow, the CD34⁺ cells are a mixture of stem cells, progenitors, and white blood cells of various degrees of maturity. Of the cells collected, between 5 and 20 percent will be true HSC. Accordingly, there is a need in the art for increasing the number of HSC in the bloodstream. The present invention addresses this need 1 by disclosing a composition for use in increasing the number of HSC in the bone marrow and increasing the mobilization of these HSC to migrate from the bone marrow to the peripheral bloodstream.

### SUMMARY OF THE INVENTION

The invention can be defined by the appending claims. Provided herein is a composition for use in increasing the mobility of a HSC population. The composition is to be administered to a mammal in need thereof. The composition may increase the number of HSC in bone marrow. The composition increases the mobility of HSC from the bone marrow to the blood stream. The stem cell population may comprise long-term HSC or committed progenitor cells.

The composition comprises a lipopeptide. The lipopeptide is a compound of the formula: 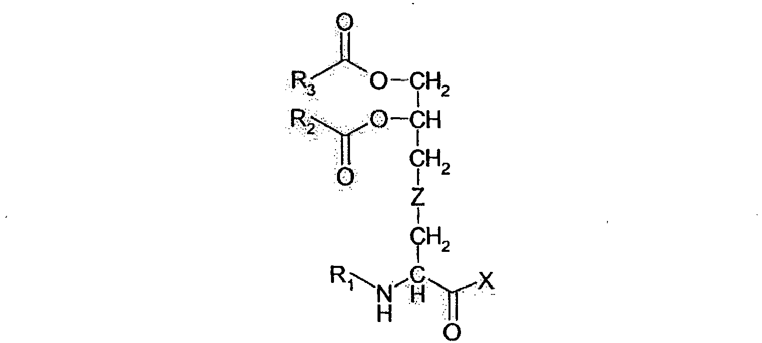 wherein,
R₁ represents H or -CO-R₄,
R₂, R₃ and R₄ independently are H or optionally substituted C₈-C₁₆ aliphatic;
X is a peptide; and
Z is S or CH₂.

The peptide is selected from the group consisting of SEQ ID NOS: 8, 16-18, 20, and 21. R₁ may be H, and R₂ and R₃ may be C₁₆ aliphatics or substitutions thereof. The compound may be an RR or RS stereoisomer, or mixture thereof.

A method for increasing the mobility of a HSC population is described herein. The method comprises administering the above composition to a mammal in need thereof.

The method may comprise administering the agent in combination with a G-CSF receptor agonist to a mammal in need thereof. The method may comprise administering the agent in combination with G-CSF. The method may comprise administering the agent in combination with a CXCR4 antagonist. The method may comprise administering the agent with AMD3100. The method may comprise administering the agent in combination with G-CSF and a CXCR4 antagonist. The method may comprise administering the agent in combination with G-CSF and AMD3100. The method may comprise administering the agent in combination with G-CSF, AMD3100, and a CXCR4 antagonist. The agent may be administered before, simultaneously with, or after administering G-CSF. The agent may be administered hourly prior to, or after, administering G-CSF. The agent may begin to be administered at anytime within 96 hours prior to, or after, G-CSF administration. The method may further comprise harvesting peripheral blood mononuclear cells from the mammal.

The method may further comprise administering a radioprotectant, which may be an antioxidant, flagellin, TGFβ, activator of a TLR, or cytokine. The cytokine may be a stem cell factor.

The method may also treat an effect of an abnormal condition comprising increasing the HSC population cell number and mobility to the bloodstream according to a method described herein. The abnormal condition may be selected from the group consisting of radiation, wounds, poisoning, infection, autologous bone marrow transplant, and bone marrow failure.

A method of treating a disease is described herein. The disease may be associated with hematopoietic and/or lymphoid injury. The disease may be a malignant or non-malignant hematological disorder, severe combined immune deficiency (SCID), Wiskott-Aldrich syndrome, or Chediak-Higashi syndrome. The method may comprise increasing a HSC population cell number and mobility to the bloodstream according to a method described herein. The disease may be selected from the group consisting of cancer, an autoimmune disease, an immunosuppression disease, anemia, thalassemia, and sickle cell anemia. The cancer may be selected from the group consisting of breast cancer, testicular cancer, neuroblastoma, ovarian cancer, leukemia, lymphoma, myeloma, and Waldenstrom's Macroglobulinemia. The autoimmune disease may be an HIV infection.

Also provided herein is a composition comprising a lipopeptide for use in treating a disease in a mammal in need thereof, wherein the lipopeptide is a compound of the formula: 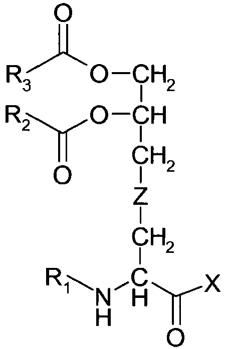 wherein,
R₁ represents H or -CO-R₄,
R₂, R₃ and R₄ independently are H or optionally substituted C₈-C₁₆ aliphatic;
X is a peptide selected from the group consisting of SEQ ID NOs: 8, 16-18, 20, and 21;
Z is S or CH₂; and
wherein the disease is selected from the group consisting of cancer, an autoimmune disease, an anemia, a non-malignant hematological disorder, Wiskott-Aldrich syndrome, Chediak-Higashi syndrome, Severe Combined Immune Deficiency (SCID), thalassemia, and sickle cell anemia. The cancer may be a cancer selected from the group consisting of leukemia, lymphoma, a malignant hematological disorder, breast cancer, testicular cancer, neuroblastoma, ovarian cancer, myeloma, and Waldenstrom's Macroglobulinemia.

Also provided herein is a method of producing a stem cell population comprising:
(a) administering to a mammal in need thereof a composition comprising a lipopeptide, wherein the lipopeptide is a compound of the formula: 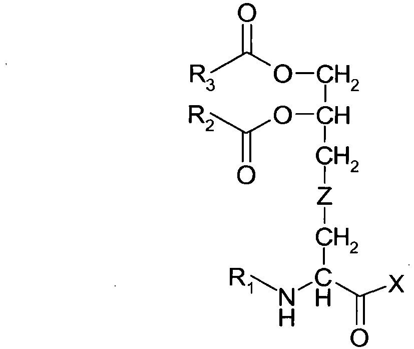 wherein,
   R₁ represents H or -CO-R₄,
   R₂, R₃ and R₄ independently are H or optionally substituted C₈-C₁₆ aliphatic;
   X is a peptide selected from the group consisting of SEQ ID NOs: 8, 16-18, 20, and 21; and
   Z is S or CH₂; and
(b) isolating peripheral white blood cells from the mammal,
wherein the peripheral white blood cells comprise the stem cell population.

Also described herein is a method of treating an effect of a cancer treatment, which may comprise administering an autologous stem cell population to a mammal that has received a cancer treatment. The cancer treatment may be chemotherapy or radiation therapy. The autologous stem cell population may be administered by injection or transfusion.

Also described herein is a method for isolating a HSC from a blood sample. The method comprises administering a composition comprising an agent to a human in need thereof The method may also comprise isolating a HSC from the sample. The agent is a lipopeptide as described herein.

### BRIEF DESCRIPTION OF THE DRAWINGS

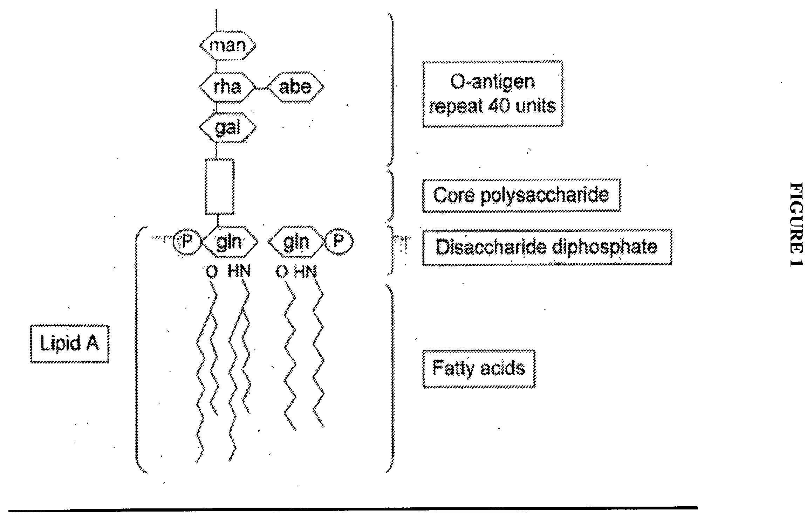

Figure 1 represents the structure of a lipopolysaccharide.
Figure 2 represents the effects of lipopeptide CBLB601 on mouse spleen weight and body weight of 6Gy of total body irradiation (TBI).
Figure 3 represents the HSC status in bone marrow. The percent of bone marrow cells that are HSC in ICR mice treated with CLBLB601 versus a control.
Figure 4 represents the hematopoietic status of stem cells in the blood wherein the number of HSC per 1 mL of blood in ICR mice treated with CBLB601 versus a control.
Figure 5 summarizes FACS data of bone marrow and blood samples isolated from a control rhesus monkey treated with G-CSF compared to monkeys treated with 0.25 mg/kg CBLB612.
Figure 6 represents FACS data of bone marrow and blood samples isolated from either control rhesus monkeys or monkeys treated with 0.25 mg/kg CBLB612 (a lipopeptide).
Figure 7 represents data related to kinetic changes in amount of cells with stem cell phenotype in blood of mice after single injection of CBLB612 followed by AMD3100.
Figure 8 represents data related to kinetic changes in amount of cells with progenitor phenotype in blood of mice after single injection of CBLB612 followed by AMD3100.
Figure 9 represents data related to kinetic changes in amount of cells with stem cell phenotype in bone marrow of mice after single injection of CBLB612 followed by AMD3100.
Figure 10 represents data related to kinetic changes in amount of cells with progenitor phenotype in bone marrow of mice after single injection of CBLB612 followed by AMD3100.
Figure 11 represents data related to kinetic changes in amount of cells with stem cell phenotype in blood of mice after single injection of CBLB612 followed by AMD3100.
Figure 12 represents data related to kinetic changes in amount of cells with progenitor phenotype in blood of mice after single injection of CBLB612 followed by AMD3100.
Figure 13 represents data related to kinetic changes in amount of cells with stem cell phenotype in bone marrow of mice after single injection of CBLB612 followed by AMD3100.
Figure 14 represents data related to kinetic changes in amount of cells with stem cell phenotype in bone marrow of mice after single injection of injection of CBLB612 followed by AMD3100.
Figure 15 represents data related to kinetic changes in amount of cells with progenitor phenotype in bone marrow of mice after single injection of CBLB612 followed by AMD3100.

### DETAILED DESCRIPTION

The invention can be defined by the appending claims. Also described herein is a method for increasing the mobility of HSC to the bloodstream. The method comprises administering a composition having an agent, which may cause the number of HSC to increase in the bone marrow, peripheral blood and umbilical cord blood. The agent increases the mobility of the HSC to migrate from the bone marrow to the bloodstream. The agent is a lipopeptide as defined in the claims. A radioprotectant or a HSC co-stimulant may be administered with the agent.

Described herein is also a method for isolating a HSC from a blood sample. The method may comprise administering a composition having an agent that increases or enhances the number of HSC and mobilizes the cells to the blood. Other aspects of the invention will become apparent to the skilled artisan by the following description of the invention.

### 1. Definitions

It is to be understood that the terminology used herein is for the purpose of describing particular embodiments only and is not intended to be limiting. It must be noted that, as used in the specification and the appended claims, the singular forms "a," "an" and "the" include plural referents unless the context clearly dictates otherwise.

The term "administer", as used herein to describe the dosage of an agent may mean a single dose or multiple doses of the agent.

The term "aliphatic" as used herein may refer to an unbranched, branched or cyclic hydrocarbon group, which may be substituted or unsubstituted, and which may be saturated or unsaturated, but which is not aromatic. The term aliphatic may include aliphatic groups, which comprise oxygen, nitrogen, sulfur or phosphorous atoms replacing one or more carbons of the hydrocarbon backbone.

The term "alkyl" as used herein alone or in combination may refer to a branched or unbranched, saturated aliphatic group. Representative examples of alkyl groups may include methyl, ethyl, n-propyl, isopropyl, n-butyl, isobutyl, sec-butyl, tert-butyl, octyl, decyl, tetradecyl, hexadecyl, eicosyl, tetracosyl and the like.

The term "alkenyl" as used herein alone or in combination may refer to a branched or unbranched, unsaturated aliphatic group containing at least one carbon-carbon double bond which may occur at any stable point along the chain. Representative examples of alkenyl groups may include ethenyl, E- and Z-pentenyl, decenyl and the like.

The term "alkynyl" as used herein alone or in combination may refer to a branched or unbranched, unsaturated aliphatic group containing at least one carbon-carbon triple bond which may occur at any stable point along the chain. Representative examples of alkynyl groups may include ethynyl, propynyl, propargyl, butynyl, hexynyl, decynyl and the like.

The term "analog" when used in the context of a peptide or polypeptide, may mean a peptide or polypeptide comprising one or more non-standard amino acids or other structural variations from the conventional set of amino acids.

The term "antibody" as used herein may mean an antibody of classes IgG, IgM, IgA, IgD or IgE, or fragments or derivatives thereof, including Fab, F(ab')₂, Fd, and single chain antibodies, diabodies, bispecific antibodies, bifunctional antibodies and derivatives thereof. The antibody may be a monoclonal antibody, polyclonal antibody, affinity-purified antibody, or mixtures thereof, which exhibit sufficient binding specificity to a desired epitope or a sequence, derived therefrom. The antibody may also be a chimeric antibody. The antibody may be derivatized by the attachment of one or more chemical, peptide, or polypeptide moieties known in the art. The antibody may be conjugated with a chemical moiety.

The term "apoptosis" as used herein may refer to a form of cell death that includes progressive contraction of cell volume with the preservation of the integrity of cytoplasmic organelles; condensation of chromatin (i.e., nuclear condensation), as viewed by light or electron microscopy; and/or DNA cleavage into nucleosome-sized fragments, as determined by centrifuged sedimentation assays. Cell death occurs when the membrane integrity of the cell is lost (e.g., membrane blebbing) with engulfment of intact cell fragments ("apoptotic bodies") by phagocytic cells.

The term "cancer" as used herein may mean any condition characterized by resistance to apoptotic stimuli.

The term "cancer treatment" as used herein may mean any treatment for cancer known in the art including, but not limited to, chemotherapy and radiation therapy.

The term "combination with" as used herein may mean that the agent may be administered prior to, together with, or after the additional treatment, or a combination thereof.

The term "derivative" when used in the context of a peptide or polypeptide, may mean a peptide or polypeptide different other than in primary structure (amino acids and amino acid analogs). By way of illustration, derivatives may differ by being glycosylated, one form of post-translational modification. For example, peptides or polypeptides may exhibit glycosylation patterns due to expression in heterologous systems. If at least one biological activity is retained, then these peptides or polypeptides are derivatives according to the invention. Other derivatives may include fusion peptides or fusion polypeptides having a covalently modified N- or C-terminus, PEGylated peptides or polypeptides, peptides or polypeptides associated with lipid moieties, alkylated peptides or polypeptides, peptides or polypeptides linked via an amino acid side-chain functional group to other peptides, polypeptides or chemicals, and additional modifications as would be understood in the art.

The term "fragment" when used in the context of a peptide or polypeptide, may mean a peptide of from about 6 to about 10 amino acids in length. The fragment may be 6, 7, 8, 9 10, 11, 12, 13, 14, 15, 16, 17, 18, 19, 20, 21, 22, 23, 24, and 25 amino acids in length.

The term "hematopoietic stem cell" or "HSC" used herein may refer to a stem cell that may be isolated from bone marrow, peripheral blood, umbilical cord blood, or embryonic stem cells. HSC may be capable of forming blood cells of the lymphoid, myeloid, and erythroid lineages. HSC may be capable of forming cells such as erythrocytes (red blood cells), platelets, granulocytes (such as neutrophils, basophils, and eosinophils), macrophages, B-lymphocytes, T-lymphocytes, and Natural killer cells. HSC may be capable of self-renewal or remaining a stem cell after cell division. HSC may also be capable of differentiation or starting a path to becoming a mature hematopoietic cell. HSC may also be regulated in their mobility or migration or may be regulated by apoptosis or programmed cell death. HSC may also be capable of differentiating into cells such as muscle (skeletal myocytes and cardiomyocytes), brain, liver, skin, lung, kidney, intestinal, and pancreatic. HSC may also be as described in Stem Cell Information [World Wide Web site]. Bethesda, MD: National Institutes of Health, U.S. Department of Health and Human Services, 2006 [cited Monday, January 08, 2007], Retrieved from http://stemcells.nih.gov/info/scireport/2006report, the contents of which are incorporated herein by reference.

The term "homolog" when used in the context of a peptide or polypeptide, may mean a peptide or polypeptide sharing a common evolutionary ancestor.

The term "saturated" as used herein may refer to a group where all available valence bonds of the backbone atoms are attached to other atoms.

The term "stem cell" used herein may refer to any cell that has the ability to divide for indefinite periods of time and to give rise to specialized cells. Stem cells may emanate from all germinal layers (i.e., ectoderm, mesoderm, and endoderm). Typical sources of stem cells may include "non-human" embryos, bone marrow, peripheral blood, umbilical cord blood, placental blood, and adipose tissue. Stem cells may be pluripotent, meaning that they are capable of generating most tissues on an organism. For example, pluripotent stem cells can give arise to cells of the skin, liver, blood, muscle, bone, etc. In contrast, multipotent or adult stem cells typically give rise to limited types of cells. Viable cells are cells that are alive and frequently are capable of growth and division. Those of skill in the art are aware of methods to determine the viability of cells, e.g., by the ability to exclude trypan blue dye. The term stem cell as used herein includes progenitor cells unless otherwise noted.

The term "substituted" as used herein may refer to a group having one or more hydrogens or other atoms removed from a carbon and replaced with a further group. Substituted groups herein may be substituted with one to five, or one to three substituents. Representative examples of such substituents include, but are not limited to aliphatic groups, aromatic groups, alkyl, alkenyl, alkynyl, aryl, alkoxy, halo, aryloxy, carbonyl, acryl, cyano, amino, nitro, phosphate-containing groups, sulfur-containing groups, hydroxyl, alkylcarbonyloxy, arylcarbonyloxy, alkoxycarbonyloxy, aryloxycarbonyloxy, alkylcarbonyl, arylcarbonyl, alkoxycarbonyl, aminocarbonyl, alkylaminocarbonyl, dialkylaminocarbonyl, alkylthiocarbonyl, acylamino, amidino, imino, alkylthio, arylthio, thiocarboxylate, alkylsulfinyl, trifluoromethyl, azido, heterocyclyl, alkylaryl, heteroaryl, semicarbazido, thiosemicarbazido, maleimido, oximino, imidate, cycloalkyl, cycloalkylcarbonyl, dialkylamino, arylcycloalkyl, arylcarbonyl, arylalkylcarbonyl, arylcycloalkylcarbonyl, arylphosphinyl, arylalkylphosphinyl, arylcycloalkylphosphinyl, arylphosphonyl, arylalkylphosphonyl, arylcycloalkylphosphonyl, arylsulfonyl, arylalkylsulfonyl, arylcycloalkylsulfonyl, combinations thereof, and substitutions thereto.

The term "treat" or "treating" when referring to protection of a mammal from a condition, may mean preventing, suppressing, repressing, or eliminating the condition. Preventing the condition involves administering a composition described herein to a mammal prior to onset of the condition. Suppressing the condition may involve administering a composition described herein to a mammal after induction of the condition but before its clinical appearance. Repressing the condition may involve administering a composition described herein to a mammal after clinical appearance of the condition such that the condition is reduced or maintained. Elimination the condition may involve administering a composition described herein to a mammal after clinical appearance of the condition such that the mammal no longer suffers the condition.

The term "unsaturated" as used herein may refer to a group where at least one available valence bond of two adjacent backbone atoms is not attached to other atoms.

The term "unsubstituted" as used herein may refer to a group that does not have any further groups attached thereto or substituted therefor.

The term "variant" when used in the context of a peptide or polypeptide, may mean a peptide or polypeptide that differs in amino acid sequence by the insertion, deletion, or conservative substitution of amino acids, but retains at least one biological activity. For purposes of this invention, "biological activity" may include the ability to be bound by a specific antibody. A conservative substitution of an amino acid, *i.e.,* replacing an amino acid with a different amino acid of similar properties (e.g., hydrophilicity, degree and distribution of charged regions) is recognized in the art as typically involving a minor change. These minor changes can be identified, in part, by considering the hydropathic index of amino acids, as understood in the art (Kyte et al., J. Mol. Biol. 157:105-132, 1982). The hydropathic index of an amino acid is based on a consideration of its hydrophobicity and charge. It is known in the art that amino acids of similar hydropathic indexes can be substituted and still retain protein function. In one aspect, amino acids having hydropathic indexes of ±2 are substituted. The hydrophilicity of amino acids can also be used to reveal substitutions that would result in proteins retaining biological function. A consideration of the hydrophilicity of amino acids in the context of a peptide permits calculation of the greatest local average hydrophilicity of that peptide, a useful measure that has been reported to correlate well with antigenicity and immunogenicity in U.S. Patent No. 4,554,101, incorporated herein by reference. Substitution of amino acids having similar hydrophilicity values can result in peptides retaining biological activity, for example immunogenicity, as is understood in the art. In one aspect, substitutions are performed with amino acids having hydrophilicity values within ±2 of each other. The particular side chain of that amino acid influences both the hydrophobicity index and the hydrophilicity value of amino acids. Consistent with that observation, amino acid substitutions that are compatible with biological function are understood to depend on the relative similarity of the amino acids, and particularly the side chains of those amino acids, as revealed by the hydrophobicity, hydrophilicity, charge, size, and other properties.

### 2. Method for Enhancing or Increasing a Characteristic of HSC Population

Provided herein is a composition for use in increasing the mobility of hematopoetic stem cells, wherein the composition is to be administered to a mammal in need thereof, and is a composition as defined in the appending claims. Also described herein is a method for increasing or enhancing a characteristic of a HSC population. The characteristic of the HSC population is cell mobility.

### a. Cell Number

Cell number may be the overall size of the HSC population. Cell number may also be the total number of individual HSC. Cell number may be enhanced or increased by increasing the rate of division of HSC into additional HSC, which may enhance and increase the total number of HSC in the bone marrow, the bloodstream, the umbilical cord, or the embryo. Cell number may also be enhanced or increased by raising the overall quantity of the HSC population, or by enhancing or increasing the amounts, numbers, or levels of the HSC population.

### b. Cell Mobility

Cell mobility may be enhanced or increased by increasing the number of individual HSC that migrate from the bone marrow into the peripheral blood. Cell mobility may also be enhanced or increased by increasing the number of individual HSC that migrate from the bone marrow to the peripheral blood, and then from the peripheral blood to particular tissues or organs such as the lymph nodes, the heart, the lung, the liver, the skin, the spleen, small and large intestines, the stomach, or the pancreas. HSC may migrate from the bone marrow to the blood stream.

Increasing the number or mobility of cells of the HSC population may also increase the rate of differentiation of the HSC population into various cell lineages. The HSC may also be capable of differentiation or starting a path to becoming a mature hematopoietic cell. For example, the cell differentiation of the HSC population may lead to an increase in the number of common myeloid progenitor cells in the bone marrow or the peripheral blood. The cell differentiation of the HSC population may also lead to an increase in the number of granulocyte/macrophage progenitor cells or megakaryocyte/erthrocyte progenitor cells in the bone marrow or peripheral blood. The HSC may differentiate into a common lymphoid precursor. The increase number of common myeloid progenitor cells may lead differentiation into granulocyte/macrophage progenitor cells or megakaryocyte/erythrocyte progenitor cells. The granulocyte/macrophage progenitor cells may further differentiate into granulocytes such as neutrophils, eosinophils, basophils, tissue precursor cells, monocytes, and immature dendritic cells. The megakaryocyte/erythrocyte progenitor cells may differentiate into megakaryocytes, erthroblast. The common lymphoid precursor cell may differeniate into B lymphocyte cells and T lymphocyte cells. The B lymphocyte cells may differentiate into antibody-secreting cells, wherein T lymphocytes may differentiate into effector T cells. The granulocyte may further differentiate into tissue mast cells, macrophages, and immature dendritic cells. The megakaryocyte may differentiate into platelets. The erythroblast may differentiate into erythrocytes. HSC may also be capable of differentiating into cells such as muscle (skeletal myocytes and cardiomyocytes), brain, liver, skin, lung, kidney, intestinal, and pancreatic. The number or proportion of cells presenting particular molecular or cell surface markers may be indicative of an HSC or HSC population.

HSC may be capable of self-renewal or remaining a stem cell after cell division. HSC may also be regulated in their migration or by apoptosis or programmed cell death. HSC may also be capable of differentiating into cells such as muscle (skeletal myocytes and cardiomyocytes), brain, liver, skin, lung, kidney, intestinal, and pancreatic.

Although not bound by theory, 1 in every 10,000 to 15,000 bone marrow cells may normally be a stem cell. In the bloodstream, the proportion may fall to 1 to 100,000 blood cells. Administering the agent in vivo may increase the number of all stem cell populations in the bloodstream in about 1 hr, 2 hrs, 3 hrs, 4 hrs, 6 hrs, 8 hrs, 10 hrs, 12 hrs, 14 hrs, 16 hrs, 18 hrs, 20 hrs, 22 hrs, 24 hrs, 26 hrs, 28 hrs, or 30 hours after infection and accumulation of stem cells including HSC in the blood may peak in about 65 hrs, 66 hrs, 67 hrs, 68 hrs, 69 hrs, 70 hrs, 71 hrs, 72 hrs, 73 hrs, 74 hrs, 75 hrs, 76 hrs, 77 hrs, 78 hrs, 79 hrs, 80 hrs, 81 hrs, 82 hrs, 83 hrs, 84 hrs, 85 hrs, 86 hrs, 87 hrs, 88 hrs, 89 hrs, 90 hrs, 91 hrs, 92 hrs, 93 hrs, 94 hrs, 95 hrs, 96 hrs, 97 hrs, 98 hrs, 99 hrs, 100 hrs, 101 hrs, 102 hrs, 103 hrs, 104 hrs, 105 hrs, 106 hrs, 107 hrs, 108 hrs, 109 hrs, and 110 hrs after administration.

### 3. Composition

The described method comprises administering to a mammal in need thereof a composition to enhance or increase the characteristic of a HSC population. The composition may also be used for the treatment of abnormal conditions associated with exposure to radiation, side effect from cancer treatments, stress, cell aging and disease. The composition may be a pharmaceutical composition, which may be produced using methods well known in the art. The composition comprises a lipopeptide as defined in the claims and hereunder. The composition may also comprise a radioprotectant, a co-stimulant of HSC, an exogenous growth factor, a cytokine or a combination thereof.

### a. Agent

The agent is a lipopeptide as defined in the claims and hereunder.

The lipopeptide is a compound of the formula: 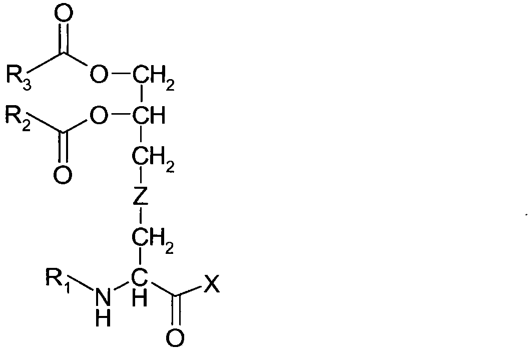 wherein,
R₁ represents H or -CO-R₄,
R₂, R₃ and R₄ independently are H or optionally substituted C₈-C₁₆ aliphatic;
X is a peptide; and
Z is S or CH₂.

The lipopeptide may comprise two or three fatty acids. Representative examples of alkyl substituents at R₂, R₃ and R₄ include C₈, C₉, C₁₀, C₁₂, C₁₄, and C₁₆. Representative examples of alkenyl substituents at R₂, R₃ and R₄ include C_{10:1}^{D1} trans.

The peptide is selected from the group consisting of SEQ ID NOs: 8, 16-18, 20, and 21.

**Table 1**

| **Sequence** | **Length** | **SEQ ID NO** |
|---|---|---|
| SNNA | 4 | 1 |
| GSSHH | 5 | 2 |
| KQNVS | 5 | 3 |
| NNSGK | 5 | 4 |
| QPDRY | 5 | 5 |
| RPDRY | 5 | 6 |
| SEEEE | 5 | 7 |
| SKKKK | 5 | 8 |
| SNNNA | 5 | 9 |
| SPPPP | 5 | 10 |
| GQHHM | 5 | 11 |
| GQHHH | 5 | 12 |
| SSHHM | 5 | 13 |
| GSHHM | 5 | 14 |
| SQMHH | 5 | 15 |
| GETDK | 5 | 16 |
| GEESN | 5 | 17 |
| GEEDD | 5 | 18 |
| TENVKE | 6 | 19 |
| QGEESNDK | 8 | 20 |
| VQGEESNDK | 9 | 21 |
| FEPPPATTT | 9 | 22 |
| GDKYFKETE | 9 | 23 |
| GDPKHPKSF | 9 | 24 |
| GGQEKSAAG | 9 | 25 |
| GPCPGCPPC | 9 | 26 |
| PPCPGCPPC | 9 | 27 |
| DNEEKPTPEQD | 11 | 28 |
| GNGGAPAQPKG | 11 | 29 |
| FEPPPATTTKSK | 12 | 30 |
| GNNDESNISFKEK | 13 | 31 |
| GDPKHPKSFTGWVA | 14 | 32 |
| AQNPNKTNSNLDSSK | 15 | 33 |
| NKDNEAEPVTEGNAT | 15 | 34 |
| SKEGNGPDPDNAAKS | 15 | 35 |
| GDKTPSTKSAGKVENK | 16 | 36 |
| GETDKEGKIIRIFDNSF | 17 | 37 |
| SSTSENNGNGNGNGGTD | 17 | 38 |
| GNNDESNISFKEKSEEEE | 18 | 39 |
| GNNDESNISFKEKSKKKK | 18 | 40 |
| GNNDESNISFKEKSPPPP | 18 | 41 |
| SSNKSTTGSGETTTAAGT | 18 | 42 |
| CGNNDESNISFKEKSKKKK | 19 | 43 |
| GSPLSFESSVQLIVSDNSS | 19 | 44 |
| SNYAKKVVKQKNHVYTPVY | 19 | 45 |
| ADVIAKIVEIVKGLIDQFTQK | 21 | 46 |
| GAASSLTYESSVQLVVSDNSS | 21 | 47 |
| GGEPAAQAPAETPAAAAEAAS | 21 | 48 |
| GQTDNNSSQSQQPGSGTTNT | 21 | 49 |
| SGALAATSDDDVKKAATVAIVA | 22 | 50 |
| SIVSTIIEVVKTNDIVKKFKK | 22 | 51 |
| SSGGGGVAADIGAGLADALTAP | 22 | 52 |

**Table 2**

| 1 | 2 | 3 | 4 | 5 |
|---|---|---|---|---|
| D | D | A | D | D |
| E | E | D | E | E |
| F | G | E | H | H |
| G | K | G | N | K |
| K | P | H | R | M |
| Q | Q | M | S | N |
| R | R | R | T | R |
| S | S | S | | S |
| | T | T | | |

The lipopeptide may be an RR- or RS-stereoisomer, or mixture thereof, with respect to the stereochemistry of the N-terminal lipoamino acid. The lipopeptide may be water-soluble.

The lipopeptide may be compound CBLB601 or CBLB612, which both have the general formula: 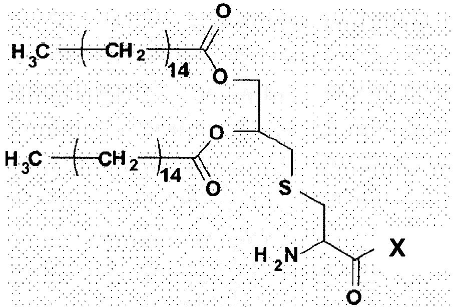 wherein X is SKKKK and VQGEESNDK, respectively.

### b. Radioprotectants

The composition described herein may also comprise a radioprotectant. The radioprotectant may be used to increase the scale of protection from ionizing radiation.

The radioprotectant may used to treat the effects of radiation exposure. The radioprotectant may be an antioxidant, a free radical scavenger, a cytokine, flagellin and latent TGFβ. The radioprotectant may also be a cytokine that may induce the expression of the cellular antioxidant proteins, such as manganese superoxide dismutase (MnSOD) and metallothionein.

The radioprotectant may also be an antioxidant and free radical scavenger that may include thiols, such as cysteine, cysteamine, glutathione and bilirubin; amifostine (WR-2721); vitamin A; vitamin C; vitamin E; and flavonoids such as Indian holy basil (Ocimum sanctum), orientin and vicenin.

The radioprotectant may be a combination of cytokines and growth factors that confer radioprotection by replenishing and/or protecting the radiosensitive stem cell populations. The radioprotectant may be cytokines that may include stem cell factor (SCF, c-kit ligand), Flt-3 ligand, interleukin-1 fragment IL-1b-rd, and keratinocyte growth factor (KGF).

The radioprotectant may also stimulate the proliferation of the immunocytes. The radioprotectant may be 5-AED (5-androstenediol), which is a steroid that stimulates the expression of cytokines, and synthetic compounds, such as ammonium tri-chloro(dioxoethylene-O,O'-) tellurate (AS-101). The radioprotectant may be latent TGFβ, flagellin and flagellin derivatives, which are strong inducers of NF-κB activity as shown in International Patent Application Nos. PCT/US2004/040656 and PCT/US2004/040753, and U.S. Patent Application No. 60/693,826.

### c. HSC Co-Stimulants

The composition as described herein may also comprise a co-stimulant of HSC. For example, the composition may be administered in conjunction with exogenous growth factors and cytokines that are specifically selected to achieve a particular outcome. The co-stimulant may be growth factors and cytokines, which stimulate proliferation and differentiation of such cell type may be used. The co-stimulant may be interleukins-1, 2, 3, 4, 5, 6, 7, 9, 10, 11, 12, 13, and 17 that are involved in lymphocyte differentiation. Interleukins 3 and 4 are involved in mast cell differentiation.

The co-stimulant may be a granulocyte macrophage colony stimulating factor (GMCSF), interleukin-3 and interleulin-5, which are involved in the eosinophil differentiation. The co-stimulant may also be a GMCSF, macrophage colony stimulating factor (MCSF) and IL-3, which are involved in macrophage differentiation. The co-stimulant may also be GM-CSF, GCSF and IL-3, which are involved in neutrophil differentiation. The co-stimulant may also be GMSCF, IL-1, IL-3, IL-6, IL-8, PIXY-321 (GM-CSF/IL-3 fusion protein), macrophage inflammatory protein, stem cell factor, thrombopoietin, growth related oncogen, IL-11 and TPO, which are involved in platelet differentiation. The co-stimulant may also be a Flt3 Ligand, which is involved in dendritic cell growth. The co-stimulant may also be a GMCSF, IL-3, and erythropoietin, which are involved in erythrocyte differentiation. The co-stimulant may be SCF, Flt3 Ligand, G-CSF, IL-3, IL-6 and IL-11, which renew primitive, pluripotent progenitor cells that are capable of sustaining hematopoiesis. The co-stimulant may be antibiotics, vitamins, herbal extracts, anti-inflammatories, glucose, antipyretics, analgesics

The composition may be used with other co-stimulants such as AMD3100 (1,1'-[1,4-phenylene-bis(methyl-ene)]-bis]1,4,8,11-tetraazacyclotetradecane) and its derivatives or other co-stimulants as disclosed in U.S. Patent No. 6,987,102. AMD3100 is an antagonist with the CXCR4 chemokine receptor. This compound may interfere with the binding of bone marrow stromal cell derived SDF-1 with CXCR4 on stem cells, which may lead to the release of HSC from bone marrow into the circulation.

### 4. Identification of Hematopoietic Stem Cells

The method may also be used to identify and isolate HSC from the bone marrow, peripheral blood, umbilical cord blood, or "non human" embryonic stem cells. The method comprises the steps of administering the lipopeptide as defined herein to a mammal or human in need thereof. The method may further comprise isolating the HSC from the blood.

Although not bound by theory, the stem cell population in the bloodstream may be heterogeneous and may include some HSC that are true, long-term self-renewing stem cells, some shorter-term progenitors, and some non-stem cells. Stem cells may also look like many other blood or bone marrow cells. The identification of HSC may require detecting the presence or absence of specific cell surface markers. A HSC may differentially express a molecular marker such as Sca-1, CD27, CD34, CD38, CD43, CD90.1, CD117 (c-Kit), AA4.1, MHC class I, CD150, Lin, CD45RO, CD45RA, CD59, CD90, CD109, CD 133, CD166, and HLA DR.

Cells surface markers characteristic of a HSC in murine and human may be different. Table 1 indicates cell surface markers that may be characteristic for mouse and human HSC as they exist in their undifferentiated state in vivo and in vitro. As HSCs begin to divide and develop into distinct cell lineages, the cell surface markers and their expression levels may change.

**Table 3 Cell-surface markers of undifferentiated HSC.**

| **Mouse** | **Human** |
|---|---|
| CD34^{low/-} | CD 34⁺ |
| SCA-1⁺ | CD59⁺ |
| CD90.1, Thy1^{+low} | Thy1⁺ |
| Flk2/flt3⁻ | CD38^{low/-} |
| CD117, C-kit⁺ | C-kit⁺ |
| lin⁻ | lin⁻ |

| | |
|---|---|
| *Lin⁻ cells lack 13 to 14 different nature blood-lineage markers* | |

For example, several marker combinations have been developed that may be indicative of murine HSC, including [CD117^{high}, CD90.1^{low}, Lin^{neg/low}, Sca-1^{pos}, Flk2/flt3⁻], [CD90.1^{low}, Lin^{neg}, Sca-1^{pos}Rhodamine123^{low}], [CD34^{neg/low}, CD117^{pos}, Sca-1^{pos}, Lin^{neg}], and "side-population" cells using Hoechst-dye. Specific detection of these combinations may allow purification of HSC to near-homogeneity.

A [CD34^{neg/low}, CD117 (c-kit) ^{pos}, Sca-1^{pos}, Lin^{neg}] may be selected via fluorescent-activated cell sorting (FACS) wherein separate several hematopoietic cell populations are simultaneously identified and isolated: A [CD117(c-kit)^{pos}, Lin^{neg}] (KL) population comprising of common myeloid progenitors (CMP); a [CD117(c-kit)^{pos}, Sca-1^{pos}, Lin^{neg}, CD34^{neg/low}] (KSL/CD34-) population consisting of long term HSC (LT-HSC); and [CD117 (c-kit)^{pos}, Sca-1^{pos}, Lin^{neg}, CD34^{pos}] (KSL/CD34⁺) population comprising short term HSC (ST-HSC) and multipotent progenitors (MPP) with sustained lymphomyelopoiesis.

The cell surface markers of HSC may also be detected using metabolic markers/dyes such as rhodamin 123, Hoeschst33342, Pyronin-Y, and BAAA. HSC may also be detected with monoclonal antibodies bearing a fluorescent label and isolated from bone marrow with FACS.

### 5. Replacement of Stressed or Dead Cells

A method for replacing cells that have been damaged or killed due to an abnormal condition or stress is also described by applying the method for increasing the characteristic of HSC. These damaged or dead cells may be blood cells of the lymphoid, myeloid, or erythroid lineages. These damaged or dead cells may be erythrocytes (red blood cells), platelets, granulocytes (such as neutrophils, basophils, and eosinophils), macrophages, B-lymphocytes, T-lymphocytes, or Natural killer cells. These damaged or dead cells may be differentiated cells such as muscle (skeletal myocytes and cardiomyocytes), brain, liver, skin, lung, kidney, intestinal, or pancreatic.

Representative examples of conditions, stresses or treatments that can cause cell damage or death may include cancer treatments, e.g., radiation therapy or chemotherapy; temperature shock; exposure to harmful doses of radiation, e.g., workers in nuclear power plants, the defense industry or radiopharmaceutical production, or duties of a soldiers; cell aging; wounding; poisoning; chemical or heat burns, viral infections, and bacterial infection.

The method replacing damaged or killed cells may be accomplished by administering to a patient in needed thereof through an autologous or heterologous treatment regimen. A return of the patient's own stem cells or a donor's stem cells to the patient may supplement or repopulate the patient's pool of HSC. The composition may be administered to the patient before or after the patient's cells have been damaged or killed. For example, the patient may be treated with the composition, and the HSC may be isolated after a time from the patient. The patient may then be exposed to the injury, stress or condition. The patient may subsequently be administered his/her own isolated HSC. Similarly, the method may be accomplished through a donor. The donor may be administered the composition. After a time, the HSC may be isolated from the donor and administered to the patient.

### a. Treatment of Cells Damaged or Killed During Cancer Treatment

The abnormal condition may be damage to normal tissue and cells attributable to cancer or cancer treatments. For example, the method may be used for providing a supply of stem cells to a patient undergoing chemotherapy. Cancer treatment to eradicate a patient's cancer cell population may eliminate the patient's bone marrow stem cells. A return of the patient's own or a donor's stored stem cells to the patient may supplement or repopulate the patient's in vivo pool of HSCs. The method may increase the number of HSC and mobilize these cells from the bone marrow to the bloodstream and may allow the use of greater doses of cancer treatments such as chemo- or radiotherapy, but with less risk than bone marrow transplantation.

### (1) Autologous or heterologous stem cell population transplant

The composition may be administered to a patient before undergoing cancer treatment or to a donor. Blood or peripheral white blood cells (PWBC), which may comprise a stem cell population comprising HSC, may be isolated from the patient or donor. The cells may be isolated from the patient after administering the composition and prior to cancer treatment. The autologous or heterologous stem cell population may be stored for future use. The stem cell population may later be administered to the patient who has previously undergone a cancer treatment. In addition, the stored autologous stem cells may be used in transplants. The composition may used in hematological bone marrow stem cell transplantation. The composition may enhance the success of transplantation before, during, and following immunosuppressive treatments. Autologous stem cell transplants may have the advantage of a lower risk of graft rejection and infection, since the recovery of immune function may be efficient. The incidence of a patient experiencing graft-versus-host disease may be very low as the donor and recipient patients are the same individual.

### (2) Chemotherapy

The cancer treatment may comprise administration of a cytotoxic agent or cytostatic agent, or combination thereof. The cytotoxic agent may prevent cancer cells from multiplying by: (1) interfering with the cell's ability to replicate DNA and (2) inducing cell death and/or apoptosis in the cancer cells. The cytostatic agent act via modulating, interfering or inhibiting the processes of cellular signal transduction that regulate cell proliferation.

Classes of compounds that may be used as cytotoxic agents include the following: alkylating agents (including, without limitation, nitrogen mustards, ethylenimine derivatives, alkyl sulfonates, nitrosoureas and triazenes): uracil mustard, chlormethine, cyclophosphamide (CYTOXAN®), ifosfamide, melphalan, chlorambucil, pipobroman, triethylene-melamine, triethylenethiophosphoramine, busulfan, carmustine, lomustine, streptozocin, dacarbazine, and temozolomide; antimetabolites (including, without limitation, folic acid antagonists, pyrimidine analogs, purine analogs and adenosine deaminase inhibitors): methotrexate, 5-fluorouracil, floxuridine, cytarabine, 6-mercaptopurine, 6-thioguanine, fludarabine phosphate, pentostatine, and gemcitabine; natural products and their derivatives (for example, vinca alkaloids, antitumor antibiotics, enzymes, lymphokines and epipodophyllotoxins): vinblastine, vincristine, vindesine, bleomycin, dactinomycin, daunorubicin, doxorubicin, epirubicin, idarubicin, ara-c, paclitaxel (paclitaxel is commercially available as TAXOL®), mithramycin, deoxyco-formycin, mitomycin-c, 1-asparaginase, interferons (preferably IFN-α), etoposide, and teniposide.

Other proliferative cytotoxic agents are navelbene, CPT-11, anastrazole, letrazole, capecitabine, reloxafine, cyclophosphamide, ifosamide, and droloxafine.

Microtubule affecting agents interfere with cellular mitosis and are well known in the art for their cytotoxic activity. Microtubule affecting agents that may be used include, but are not limited to, allocolchicine (NSC 406042), halichondrin B (NSC 609395), colchicine (NSC 757), colchicine derivatives (e.g., NSC 33410), dolastatin 10 (NSC 376128), maytansine (NSC 153858), rhizoxin (NSC 332598), paclitaxel (TAXOL®, NSC 125973), TAXOL® derivatives (e.g., derivatives (e.g., NSC 608832), thiocolchicine NSC 361792), trityl cysteine (NSC 83265), vinblastine sulfate (NSC 49842), vincristine sulfate (NSC 67574), natural and synthetic epothilones including but not limited to epothilone A, epothilone B, and discodermolide (see Service, (1996) Science, 274:2009) estramustine, nocodazole, MAP4, and the like. Examples of such agents are also described in Bulinski (1997) J. Cell Sci. 110:3055 3064; Panda (1997) Proc. Natl. Acad. Sci. USA 94:10560-10564; Muhlradt (1997) Cancer Res. 57:3344-3346; Nicolaou (1997) Nature 387:268-272; Vasquez (1997) Mol. Biol. Cell. 8:973-985; and Panda (1996) J. Biol. Chem 271:29807-29812.

Also suitable are cytotoxic agents such as epidophyllotoxin; an antineoplastic enzyme; a topoisomerase inhibitor; procarbazine; mitoxantrone; platinum coordination complexes such as cis-platin and carboplatin; biological response modifiers; growth inhibitors; antihormonal therapeutic agents; leucovorin; tegafur; and haematopoietic growth factors.

Cytostatic agents that may be used also hormones and steroids (including synthetic analogs): 17 α-ethinylestradiol, diethylstilbestrol, testosterone, prednisone, fluoxymesterone, dromostanolone propionate, testolactone, megestrolacetate, methylprednisolone, methyltestosterone, prednisolone, triamcinolone, chlorotrianisene, hydroxyprogesterone, aminoglutethimide, estramustine, medroxyprogesteroneacetate, leuprolide, flutamide, toremifene, and zoladex.

Other cytostatic agents are antiangiogenics, such as matrix metalloproteinase inhibitors, and other VEGF inhibitors, such as anti-VEGF antibodies and small molecules such as ZD6474 and SU6668 are also included. Anti-Her2 antibodies from Genentech may also be utilized. A suitable EGFR inhibitor is EKB-569 (an irreversible inhibitor). Also included are Imclone antibody C225 immunospecific for the EGFR, and src inhibitors.

Also suitable for use as a cytostatic agent is CASODEX® (bicalutamide, Astra Zeneca) which renders androgen-dependent carcinomas non-proliferative. Yet another example of a cytostatic agent is the antiestrogen TAMOXIFEN® which inhibits the proliferation or growth of estrogen dependent breast cancer. Inhibitors of the transduction of cellular proliferative signals are cytostatic agents. Representative examples include epidermal growth factor inhibitors, Her-2 inhibitors, MEK-1 kinase inhibitors, MAPK kinase inhibitors, PI3 inhibitors, Src kinase inhibitors, and PDGF inhibitors.

### (3) Radiation Therapy for Cancer

The cancer treatment may comprise radiation therapy. The radiation therapy may be external beam radiation, internal radiation therapy, or conformal radiation therapy, in which a computer is used to shape the beam of radiation to match the shape of the tumor. The radiation used in radiation therapy may come from a variety of sources, including an x-ray, electron beam, or gamma rays. The doses and timing of administration of the radiation during radiation therapy can and will vary depending on the location and extent of the cancer. The composition may be administered with a radioprotectant during radiation therapy, as described above.

### b. Chemotherapy/Radiation and/or Antiviral Therapy for Treating HIV Infection

The method may comprise administering a composition that increases the number of HSC in bone marrow and mobilizes these cells to the bloodstream to alleviate or treat the symptoms of chemotherapy or radiation therapy associated with treatment of an immune deficiency. The composition may be administered to a patient who has undergone myeloablative chemotherapy or radiotherapy for AIDS. For example, the composition may be administered to a patient to mobilize or increase the number of HSC from a patient's bone marrow. The mobilized HSC may then be collected from peripheral blood by leukapheresis. HSC may then enriched from the collected peripheralized blood by immunoadsorption using anti-CD34 antibodies. Optionally, the enriched HSC may be expanded ex vivo by culturing them in the presence of agents that stimulate proliferation of stem cells. Following administration of myeloablative chemotherapy or radiotherapy, the enriched, and optionally expanded HSC may then be returned to the patient's circulating blood and allowed to engraft themselves into the bone marrow.

In addition, this method further optionally involves administration to the patient of anti-HIV compounds, such as antivirals such as AZT, soluble CD4, and CD4-directed blockers of the AIDS virus or antisense or antigene oligonucleotides, both before and after the return of the enriched and optionally expanded HSC to the patient's circulating blood. This step serves a "mopping up" function to prevent residual virus from infecting the progeny of the newly returned stem cells.

### c. Modulation of Cell Aging

The method described herein may increase the number of HSC and mobilize these cells to the bloodstream. Autologous stem cells may be isolated from blood or peripheral white blood cells and administered for replacing stressed or dead cells due to cell aging.

### d. Radiation

The method described herein may replace stressed or dead cells attributable to radiation exposure. The method may also replace stressed or damaged cells due to radiation therapy.

Exposure to ionizing radiation (IR) may be short- or long-term, it may be applied as a single dose or multiple doses, to the whole body or locally. Thus, nuclear accidents or military attacks may involve exposure to a single high dose of whole body irradiation (sometimes followed by a long-term poisoning with radioactive isotopes). Likewise, a single dose of radiation is generally used for the pretreatment of bone marrow transplant patients when it is necessary to prepare the host's hematopoietic organs for the donor's bone marrow by "cleaning" them from the host blood precursors.

At the molecular and cellular level, radiation particles may lead to breakage in the DNA and cross-linking between DNA, proteins, cell membranes and other macromolecular structures. Ionizing radiation may also induce secondary damage to the cellular components by giving rise to free radicals and reactive oxygen species (ROS). Multiple repair systems counteract this damage, such as several DNA repair pathways that restore the integrity and fidelity of the DNA, and antioxidant chemicals and enzymes that scavenge the free radicals and ROS and reduce the oxidized proteins and lipids. Cellular checkpoint systems are present to detect the DNA defects and delay cell cycle progression until the damage is repaired or a decision to commit the cell to growth arrest or programmed cell death (apoptosis) is reached.

At the organism level, the immediate effects of low and moderate levels of radiation are largely caused by cell death, which leads to radiation-induced inflammation. At higher radiation levels, the so-called hematopoietic and gastrointestinal syndromes lead to short-term radiation-induced death. The hematopoietic syndrome is characterized by the loss of hematopoietic cells and their progenitors, thereby making it impossible to regenerate blood and the lymphoid system. Death usually occurs as a consequence of infection (due to immunosuppression), hemorrhage and/or anemia. The gastrointestinal syndrome is characterized by massive cell death in the intestinal epithelium, predominantly in the small intestine, followed by the disintegration of the intestinal wall and death from bacteriemia and sepsis. The hematopoietic syndrome manifests itself at lower doses of radiation and leads to a more delayed death than the gastrointestinal syndrome. Very high doses of radiation can cause nearly instant death by eliciting neuronal degeneration.

The method described herein may also increase the scale of protection from ionizing radiation.

### e. Burn Treatment

The method may comprise administering a composition that increases the number of HSC in bone marrow and mobilizes these cells to the bloodstream to alleviate or treat a burn. The method may comprise increasing a characteristic of a HSC population and isolating peripheral white blood cells. The peripheral white blood cells may comprise autologous stem cells, which may be administered to a burned mammal. The autologous stem cells may be a source of trophic factors that promote tissue regeneration at the burn. The burn may be associated with exposure to radiation.

### 6. Treatment of Diseases

A method for treating a disease with HSC is also described. The method may comprise administering a composition that increases the number of HSC in bone marrow and mobilizes these cells to the bloodstream to replace diseased cells.

### a. Repopulating Hemopoietic Cell Pools

The composition may be used in a method of treating a disease or condition for which repopulating the erythroid, myeloid, or lymphoid hematopoietic cell pool is indicated. For example, the composition may be used in a method of treating a disease or condition when HSC transplantation such as bone marrow transplantation into an animal or human is indicated. The composition may be used to restore or prevent a deficiency in hematopoietic cell number in a subject. The deficiency may arise, for example, from a benign disease or condition, genetic abnormality, disease, stress, chemotherapy, or from radiation treatment.

This method involves the same steps as described for transplanting mobilized HSC into a patient who has undergone chemotherapy or radiotherapy for AIDS as described above.

### b. Cancer Treatment

The method may comprise administering a composition that increases the number of HSC in bone marrow and mobilize these cells to migrate to the bloodstream to alleviate or treat cancer. The cancer may be a hematologic malignancy including, without limitation, hematopoietic tumors of lymphoid lineage including leukemia, acute lymphocytic leukemia, acute lymphoblastic leukemia, B-cell lymphoma, T-cell lymphoma, Hodgkins lymphoma, non-Hodgkins lymphoma, hairy cell lymphoma, histiocytic lymphoma, and Burketts lymphoma; hematopoietic tumors of myeloid lineage including acute and chronic myelogenous leukemias, myelodysplastic syndrome, myeloid leukemia, and promyelocytic leukemia. Other cancers that may be treated include the following: carcinoma including that of the bladder (including accelerated and metastatic bladder cancer), breast, colon (including colorectal cancer), kidney, liver, lung (including small and non-small cell lung cancer and lung adenocarcinoma), ovary, prostate, testes, genitourinary tract, lymphatic system, larynx, pancreas (including exocrine pancreatic carcinoma), mouth, pharynx, esophagus, stomach, small intestine, colon, rectum, gall bladder, cervix, thyroid, and skin (including squamous cell carcinoma); tumors of the central and peripheral nervous system including astrocytoma, neuroblastoma, glioma, and schwannomas; tumors of mesenchymal origin including fibrosarcoma, rhabdomyoscarcoma, and osteosarcoma; and other tumors including melanoma, xenoderma pigmentosum, keratoactanthoma, seminoma, thyroid follicular cancer, and teratocarcinoma.

### c. Blood disorders/autoimmune disease

The method may comprise administering a composition that increases the number of HSC in bone marrow and mobilizes these cells to the bloodstream to alleviate or treat the symptoms of a benign disease or condition. The benign disease or disorder may be associated with the hematopoietic system including, without limitation, a hemoglobinopathy, a bone marrow failure syndrome, an immune deficiency, a metabolic/storage disease, a neutrophil disorder, a platelet disease, a viral infection such as an HIV infection, and an autoimmune disorder. The hemoglobinopathy may be thalassemia (e.g., transfusion-dependent thalassemia, thalassemia major, etc.) or thalassemia sickle cell anemia or sickle cell disease.

### 7. Administration of Composition

Administration of the compositions using the method described herein may be orally, parenterally, sublingually, transdermally, rectally, transmucosally, topically, via inhalation, via buccal administration, or combinations thereof. Parenteral administration includes, but is not limited to, intravenous, intraarterial, intraperitoneal, subcutaneous, intramuscular, intrathecal, and intraarticular. For veterinary use, the composition may be administered as a suitably acceptable formulation in accordance with normal veterinary practice. The veterinarian can readily determine the dosing regimen and route of administration that is most appropriate for a particular animal. The compositions may be administered to a human patient, cat, dog, large animal, or an avian.

The composition may be administered simultaneously or metronomically with other treatments. The term "simultaneous" or "simultaneously" as used herein, means that the composition and other treatment be administered within 48 hours, preferably 24 hours, more preferably 12 hours, yet more preferably 6 hours, and most preferably 3 hours or less, of each other. The term "metronomically" as used herein means the administration of the composition at times different from the other treatment and at a certain frequency relative to repeat administration.

The composition may be administered at any point prior to exposure to the abnormal conditions, stress, or treatment including about 120 hr, 118 hr, 116 hr, 114 hr, 112 hr, 110 hr, 108 hr, 106 hr, 104 hr, 102 hr, 100 hr, 98 hr, 96 hr, 94 hr, 92 hr, 90 hr, 88 hr, 86 hr, 84 hr, 82 hr, 80 hr, 78 hr, 76 hr, 74 hr, 72 hr, 70 hr, 68 hr, 66 hr, 64 hr, 62 hr, 60 hr, 58 hr, 56 hr, 54 hr, 52 hr, 50hr, 48 hr, 46 hr, 44 hr, 42 hr, 40 hr, 38 hr, 36 hr, 34 hr, 32 hr, 30 hr, 28 hr, 26 hr, 24 hr, 22 hr, 20 hr, 18 hr, 16 hr, 14 hr, 12 hr, 10 hr, 8 hr, 6 hr, 4 hr, 3 hr, 2 hr, or 1 hr prior to exposure. The composition may also be administered at any point after exposure including about 1 hr, 2 hr, 3 hr, 4 hr, 6 hr, 8 hr, 10 hr, 12 hr, 14 hr, 16 hr, 18 hr, 20 hr, 22 hr, 24 hr, 26 hr, 28 hr, 30 hr, 32 hr, 34 hr, 36 hr, 38 hr, 40 hr, 42 hr, 44 hr, 46 hr, 48 hr, 50 hr, 52 hr, 54 hr, 56 hr, 58 hr, 60 hr, 62 hr, 64 hr, 66 hr, 68 hr, 70 hr, 72 hr, 74 hr, 76 hr, 78 hr, 80 hr, 82 hr, 84 hr, 86 hr, 88 hr, 90 hr, 92 hr, 94 hr, 96 hr, 98 hr, 100 hr, 102 hr, 104 hr, 106 hr, 108 hr, 110 hr, 112 hr, 114 hr, 116 hr, 118 hr, and 120 hr after exposure.

The composition may be administered to a donor prior to processing of the HSC about 120 hr, 118 hr, 116 hr, 114 hr, 112 hr, 110 hr, 108 hr, 106 hr, 104 hr, 102 hr, 100 hr, 98 hr, 96 hr, 94 hr, 92 hr, 90 hr, 88 hr, 86 hr, 84 hr, 82 hr, 80 hr, 78 hr, 76 hr, 74 hr, 72 hr, 70 hr, 68 hr, 66 hr, 64 hr, 62 hr, 60 hr, 58 hr, 56 hr, 54 hr, 52 hr, 50hr, 48 hr, 46 hr, 44 hr, 42 hr, 40 hr, 38 hr, 36 hr, 34 hr, 32 hr, 30 hr, 28 hr, 26 hr, 24 hr, 22 hr, 20 hr, 18 hr, 16 hr, 14 hr, 12 hr, 10 hr, 8 hr, 6 hr, 4 hr, 3 hr, 2 hr, or 1 hr prior to processing.

### 8. Formulation of Composition

The method may comprise administering a composition that increases the number of HSC in bone marrow and mobilizes these cells to the bloodstream to alleviate or treat the symptoms of a disease or condition. Compositions provided herein may be in the form of tablets or lozenges formulated in a conventional manner. For example, tablets and capsules for oral administration may contain conventional excipients including, but not limited to, binding agents, fillers, lubricants, disintegrants and wetting agents. Binding agents include, but are not limited to, syrup, accacia, gelatin, sorbitol, tragacanth, mucilage of starch and polyvinylpyrrolidone. Fillers include, but are not limited to, lactose, sugar, microcrystalline cellulose, maizestarch, calcium phosphate, and sorbitol. Lubricants include, but are not limited to, magnesium stearate, stearic acid, talc, polyethylene glycol, and silica. Disintegrants include, but are not limited to, potato starch and sodium starch glycollate. Wetting agents include, but are not limited to, sodium lauryl sulfate. Tablets may be coated according to methods well known in the art.

Compositions provided herein may also be liquid formulations including, but not limited to, aqueous or oily suspensions, solutions, emulsions, syrups, and elixirs. The compositions may also be formulated as a dry product for constitution with water or other suitable vehicle before use. Such liquid preparations may contain additives including, but not limited to, suspending agents, emulsifying agents, nonaqueous vehicles and preservatives. Suspending agent include, but are not limited to, sorbitol syrup, methyl cellulose, glucose/sugar syrup, gelatin, hydroxyethylcellulose, carboxymethyl cellulose, aluminum stearate gel, and hydrogenated edible fats. Emulsifying agents include, but are not limited to, lecithin, sorbitan monooleate, and acacia. Nonaqueous vehicles include, but are not limited to, edible oils, almond oil, fractionated coconut oil, oily esters, propylene glycol, and ethyl alcohol. Preservatives include, but are not limited to, methyl or propyl p-hydroxybenzoate and sorbic acid.

Compositions provided herein may also be formulated as suppositories, which may contain suppository bases including, but not limited to, cocoa butter or glycerides. Compositions provided herein may also be formulated for inhalation, which may be in a form including, but not limited to, a solution, suspension, or emulsion that may be administered as a dry powder or in the form of an aerosol using a propellant, such as dichlorodifluoromethane or trichlorofluoromethane. Compositions provided herein may also be formulated as transdermal formulations comprising aqueous or nonaqueous vehicles including, but not limited to, creams, ointments, lotions, pastes, medicated plaster, patch, or membrane.

Compositions provided herein may also be formulated for parenteral administration including, but not limited to, by injection or continuous infusion. Formulations for injection may be in the form of suspensions, solutions, or emulsions in oily or aqueous vehicles, and may contain formulation agents including, but not limited to, suspending, stabilizing, and dispersing agents. The composition may also be provided in a powder form for reconstitution with a suitable vehicle including, but not limited to, sterile, pyrogen-free water.

Compositions provided herein may also be formulated as a depot preparation, which may be administered by implantation or by intramuscular injection. The compositions may be formulated with suitable polymeric or hydrophobic materials (as an emulsion in an acceptable oil, for example), ion exchange resins, or as sparingly soluble derivatives (as a sparingly soluble salt, for example).

### 9. Dosage of Composition

The method may comprise administering a therapeutically effective amount of the composition to a patient in need thereof. The therapeutically effective amount required for use in therapy varies with the nature of the condition being treated, the length of time desired to increase HSC into the bloodstream, and the age/condition of the patient. In general, however, doses employed for adult human treatment typically are in the range of 0.001 mg/kg to about 200 mg/kg per day. The dose may be about 1 µg/kg to about 100 µg/kg per day. The desired dose may be conveniently administered in a single dose, or as multiple doses administered at appropriate intervals, for example as two, three, four or more sub-doses per day. Multiple doses may be desired, or required.

The dosage may be at any dosage including, but not limited to, about 1 µg/kg, 25 µg/kg, 50 µg/kg, 75 µg/kg, 100 µg/kg, 125 µg/kg, 150 µg/kg, 175 µg/kg, 200 µg/kg, 225 µg/kg, 250 µg/kg, 275 µg/kg, 300 µg/kg, 325 µg/kg, 350 µg/kg, 375 µg/kg, 400 µg/kg, 425 µg/kg, 450 µg/kg, 475 µg/kg, 500 µg/kg, 525 µg/kg, 550 µg/kg, 575 µg/kg, 600 µg/kg, 625 µg/kg, 650 µg/kg, 675 µg/kg, 700 µg/kg, 725 µg/kg, 750 µg/kg, 775 µg/kg, 800 µg/kg, 825 µg/kg, 850 µg/kg, 875 µg/kg, 900 µg/kg, 925 µg/kg, 950 µg/kg, 975 µg/kg or 1 mg/kg.

### 10. Gene Therapy

A method for carrying out gene therapy in patients having various genetic and acquired diseases using a composition that increases the number of HSC in bone marrow and mobilizes these cells to the bloodstream is described herein. In this method, HSC may be mobilized from a patient's bloodstream by administration of the composition. Peripheral blood is then collected by leukapheresis. HSC may be further enriched from the collected peripheral blood by immunoadsorption using anti-CD34 antibodies. Optionally, the enriched HSC are then expanded ex vivo by culturing them in the presence of agents that stimulate proliferation of stem cells. The enriched and optionally expanded stem cells are then transduced with an amphotrophic retroviral vector, or other suitable vectors, that expresses a gene that ameliorates the genetic or acquired disease. Optionally, the vector may also carry an expressed selectable marker, in which case successfully transduced cells may be selected for the presence of the selectable marker. The transduced and optionally selected HSC are then returned to the patient's circulating blood and allowed to engraft themselves into the bone marrow.

This invention has multiple aspects, illustrated by the following non-limiting examples.

### EXAMPLES

### Example 1

### Lipopeptides in Radioprotection

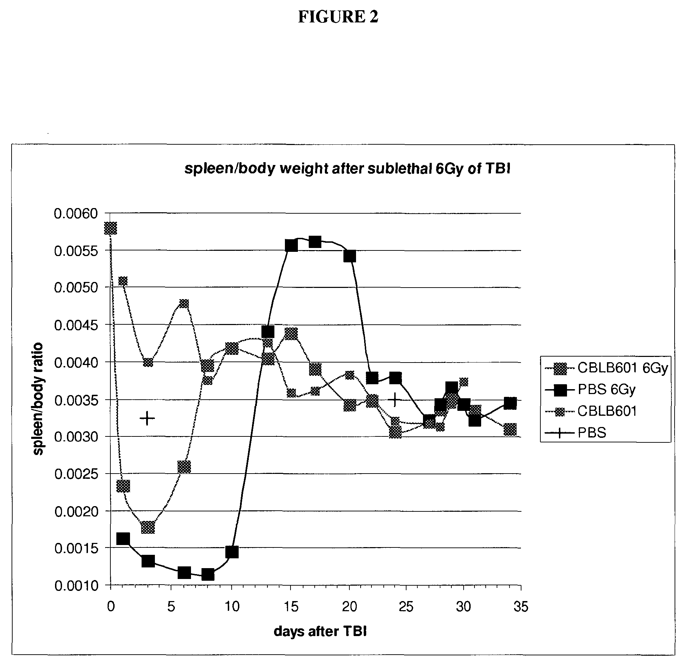

Lipopeptides are capable of protecting a mammal from the effects of radiation. This effect was demonstrated by monitoring the immune system via spleen weight following irradiation as follows. ICR mice were injected with PBS or 3 µg CBLB601, and then exposed to 0 or 6 Gy total body irradiation (TBI). Groups of five mice from each treatment were sacrificed every 3 days and their spleens and thymuses were removed and weighed. Spleen weight was normalized to body weight for each corresponding animal. Figure 2 shows a graph of the average weights of spleens from irradiated control and CBLB601-treated mice. Spleen weight per body weight is plotted for mice exposed to 0 or 6 Gy TBI following intramuscular administration of PBS or 3 µg CBLB601 per mouse 24 h prior to irradiation. Spleens of PBS-treated mice that were exposed to 6 Gy of radiation recovered to normal weight after 13-14 days. In comparison, spleens of CBLB601-treated mice exposed to the same amount of radiation recovered to normal weight by 8 days.

### Example 2

### Blood Transfused from Lipopeptide-Treated Mice is Capable of Protecting Against Radiation

Blood transfused from lipopeptide-treated mice is capable of protecting recipient mice from the effects of radiation. This was demonstrated by injecting mice daily with 50 µg lipopeptide CBLB612 for three consecutive days. To control for the effects of CBLB612, a group of mice was injected daily for four consecutive days with 100 µg/kg G-CSF, or with PBS.

Following the last injection, blood was collected from the treated mice by cardiac puncture and combined for each group (*i.e.,* PBS negative controls, G-CSF positive controls, and CBLB612 experimental). Next, peripheral white blood cells (PWBC) were fractionated, and 200 µL of fractionated PWBC were injected into the tail veins of irradiated recipient mice. The amount of transfused PWBC was equivalent to the amount from one mouse. As additional controls, one group of mice did not receive a transfusion and another group was injected with 3 x 10⁶ bone marrow cells from naive mice.

Mice from the five test groups (*i.e.,* PBS-, G-CSF-, and CBLB612-treated mouse blood transfusions, no transfusion, and bone marrow injection), previously irradiated with 9 Gy (a lethal dose for C57/B6 mice) for 2 h prior to transfusion, were observed and their survival rate assessed following transfusion. Table 4 describes the experimental results.

**Table 4**

| | **Number of Surviving Mice** | | | | | | | | | | |
|---|---|---|---|---|---|---|---|---|---|---|---|
| **Compound** | **Day 0** | **Day 21** | **Day 22** | **Day 23** | **Day 24** | **Day 25** | **Day 26** | **Day 27** | **Day 28** | **Day 29** | **Day 30** |
| | **3/23** | **4/13** | **4/14** | **4/15** | **4/16** | **4/17** | **4/18** | **4/19** | **4/20** | **4/21** | **4/22** |
| Blood from PBS treated mice | 3 | 0 | 0 | 0 | 0 | 0 | 0 | 0 | 0 | 0 | 0 |
| Blood from G-CSF treated mice | 5 | 5 | 5 | 5 | 5 | | | | | | |
| Blood from CBLB612 treated mice | 5 | 5 | 5 | 5 | 5 | | | | | | |
| Control NO INJECTION | 3 | 1 | 1 | 1 | 1 | | | | | | |
| Injected with bone marrow | 5 | 5 | 5 | 5 | 5 | | | | | | |

Mice that did not receive a transfusion or were transfused with PWBC from PBS-treated mice showed poor survival rates at Day 21 after irradiation. In contrast, all mice transfused with PWBC isolated from CBLB612-treated mice survived to Day 24 after irradiation. This survival rate equaled that of mice treated with PWBC isolated from G-CSF-treated mice and mice injected with bone marrow cells from naive mice prior to irradiation. This indicates that blood from lipopeptide-treated mice is capable of protecting against the effects of lethal levels of radiation when transfused into irradiated recipient mice.

### Example 3

### Lipopeptides are Capable of Increasing HSC Number and Mobility

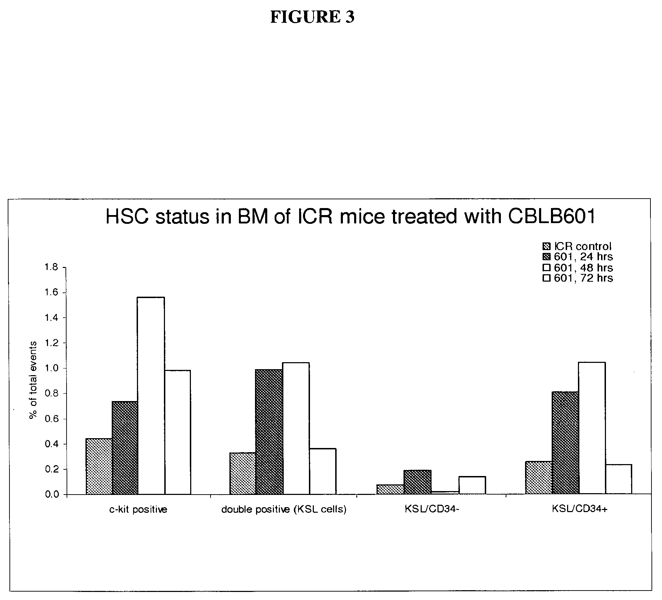

The following demonstrates that lipopeptides indeed are capable of increasing HSC number and mobilizing them from bone marrow (BM) to the blood stream. This effect was observed by monitoring known markers of HSC in BM cells and peripheral blood cells as follows. Cells were isolated from the BM of mice treated with CBLB601 or control mice, and the cells were then analyzed by fluorescence-activated cell-sorting (FACS). FACS was used to the select KL cell populations presenting CD117 (c-kit)^{pos} and Lin^{neg}, which mark common myeloid progenitors; KSL/CD34⁻ cell populations presenting CD117 (c-kit)^{pos}, Sca-1^{pos}, Lin^{neg}, and CD34^{neg/low}, which mark long-term HSC; and KSL/CD34⁺ cell populations presenting CD117 (c-kit)^{pos}, Sca-1^{pos}, Lin^{neg}, CD34^{pos}, which mark short-term HSC and multipotent progenitors with sustained lymphomyelopoiesis, but preferable reconstituting lymphopoiesis rather than myelopoiesis. Figure 3 shows that the percentages of c-kit^{pos}, KSL cells, KSL/CD34⁻, and KSL/CD34⁺ cells in BM increase in CBLB601-treated mice relative to the control and peak at 72 h after CBLB601 administration. Hence, long-term- and short-term HSC cell populations, as well as common- and multipotent myeloid progenitor cell populations are increased by CBLB601 treatment.

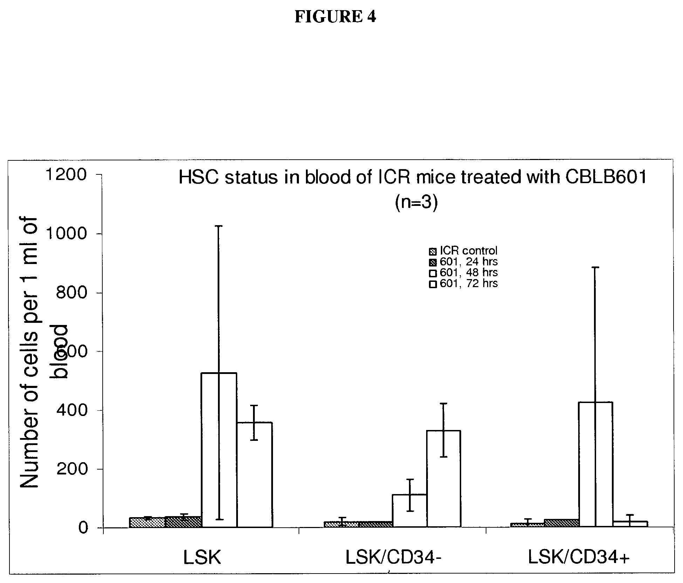

Additionally, HSC are mobilized from BM to the blood by CBLB601 treatment. FACS was used to analyze cell populations in the blood of CBLB601-treated versus control mice. Figure 4 shows that the numbers of LSK, LSK/CD34⁻, and LSK/CD34⁺ cells/mL blood increase in CBLB601-treated mice compared to a control. The increase in peripheral blood HSC cells shows a peak at 72 h after treatment with CBLB601. Accordingly, these experiments show that the radioprotective effect of lipopeptides is mediated by increased hematopoiesis.

### Example 4

### Lipopeptides are Capable of Mobilizing Multiple Stem Cell Populations

The following demonstrates that lipopeptides are capable of mobilizing multiple stem cell populations in the blood. C57/B6 mice received either one subcutaneous (SC) injection or four daily SC injections of 50 µg CBLB612. A blood sample was collected from each mouse and analyzed by FACS using the following molecular markers common among stem cell populations: Lin, sca-1, c-Kit, and CD34. Table 5 below shows the number of cells/mL for different cell populations over time in the samples as analyzed by FACS. Lin⁻, sca-1⁺, and c-Kit⁺ (LSK) are markers common to all stem cells. Cells exhibiting LSK/CD34⁻ are true pluripotent long-term hematopoietic stem cells, while cells exhibiting LSK/CD34⁺ are short-term HSC and multipotent progenitors.

**Table 5 Mobilization kinetics of hematopoietic stem to blood after CBLB612 injection (average of three animals)**

| | **C57/BL6** | **612** | **612** | **612** | **612** | **612** | **612** |
|---|---|---|---|---|---|---|---|
| | **control** | **24 h** | **48 h** | **72 h** | **96 h** | **120 h** | **4SC** |
| MNC x 10⁶/ml | 4 | 2 | 3 | 7 | 9 | 8 | 9 |
| LSK | 5 | 9 | 2 | 136 | 111 | 97 | 338 |
| LSK/CD34- | 3 | 7 | 1 | 54 | 22 | 62 | 52 |
| LSK/CD34+ | 2 | 3 | 1 | 81 | 89 | 35 | 286 |

The levels of pluripotent long-term hematopoietic stem cells, committed progenitor stem cells, and stem cells in general increased by 72 h after treatment with lipopeptide CBLB612. These data demonstrate that lipopeptides are capable of elevating levels of a broad array of stem cells, representing almost all types, and mobilizing the cells to the blood.

### Example 5

### Lipopeptides are Capable of Increasing HSC Number in the Bone Marrow and Mobility to the Peripheral Blood in Rhesus Monkeys

The following demonstrates that lipopeptides are capable of increasing HSC number and mobilizing them from bone marrow (BM) to the blood stream. This effect was observed by monitoring known markers of HSC in BM in cells and peripheral cells rhesus monkeys.

Six rhesus monkeys were studied to measure the effects of CBLB612. A positive control rhesus monkey received a daily subcutaneous dose of 100 µg/kg of G-CSF for four consecutive days prior to Day 0 (i.e., Days -4-1. On Day 1, blood was drawn from an experimental rhesus monkeys (monkeys 1-6) prior to receiving their assigned treatment. Rhesus monkeys 2-5 received 0.25 mg/kg of CBLB612 intramuscularly on Day 1. Monkey 6, a negative control (naive), received neither G-CSF nor CBLB612.

Blood was further drawn on Days 2-5 of the trial from monkeys 2-6. Bone marrow was drawn on Day 1 from monkey 6 (naïve), Day 2 from monkey 2, Day 3 from monkey 3, Day 4 from monkey 4, and on Day 5 from monkey 5 (See Table 6).

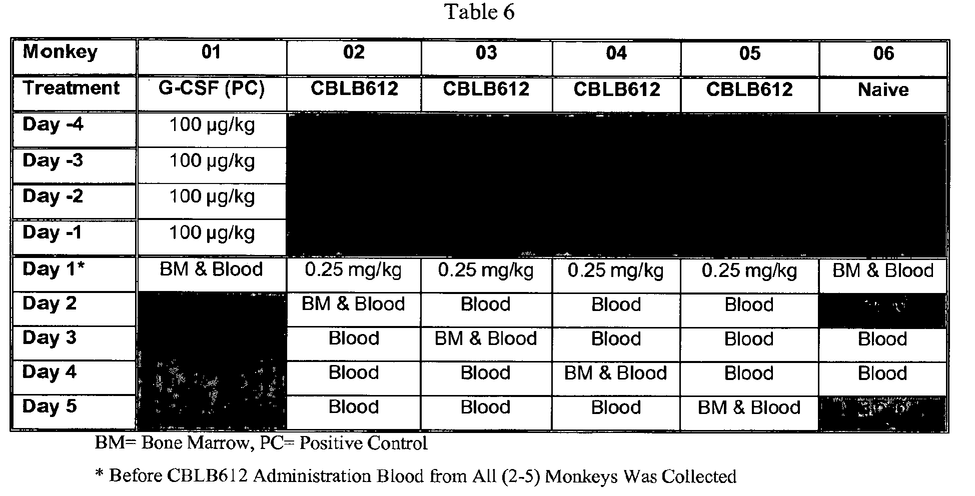

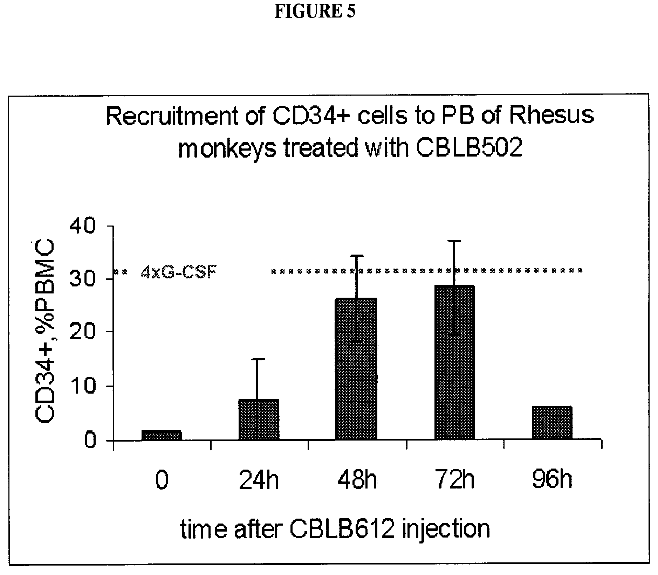

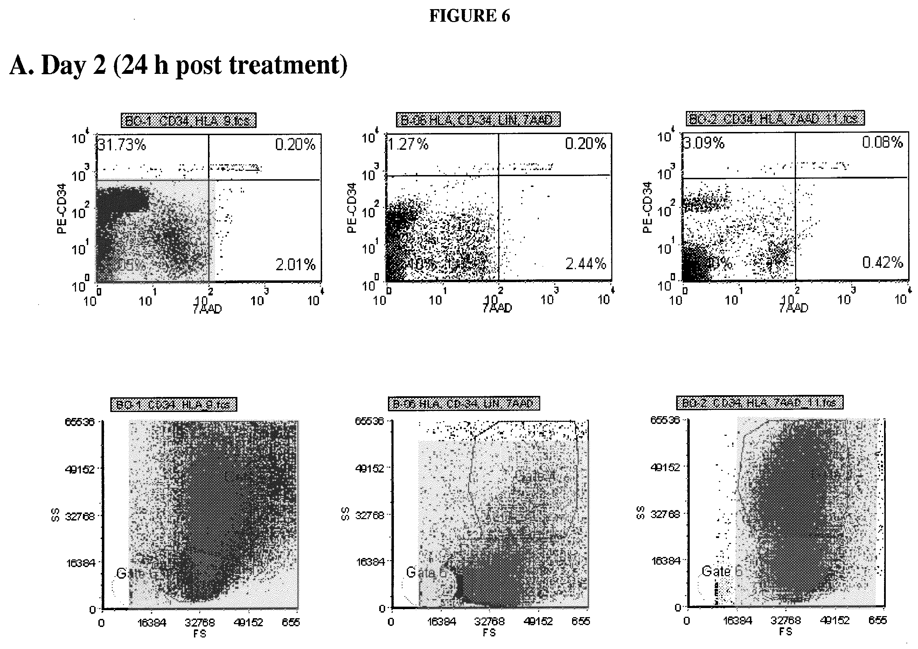

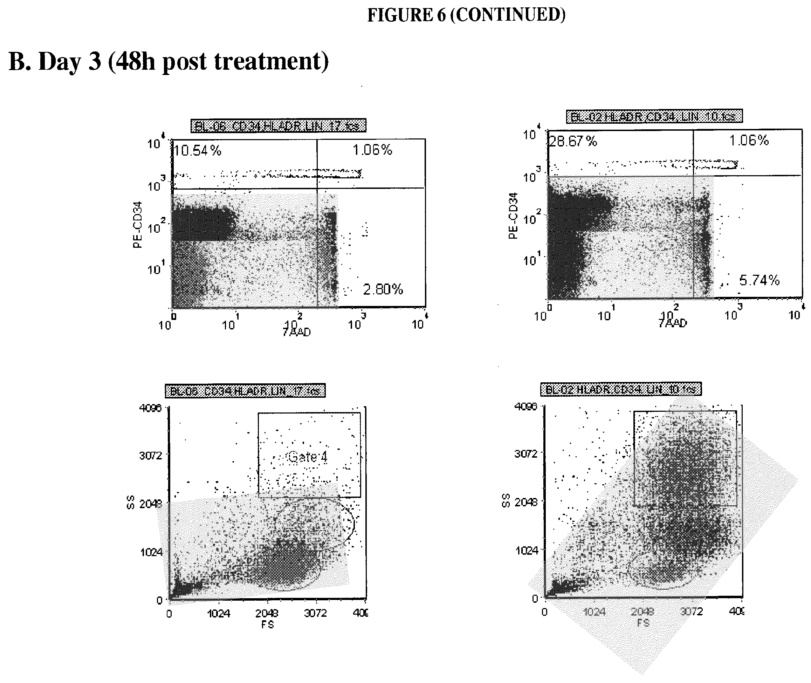

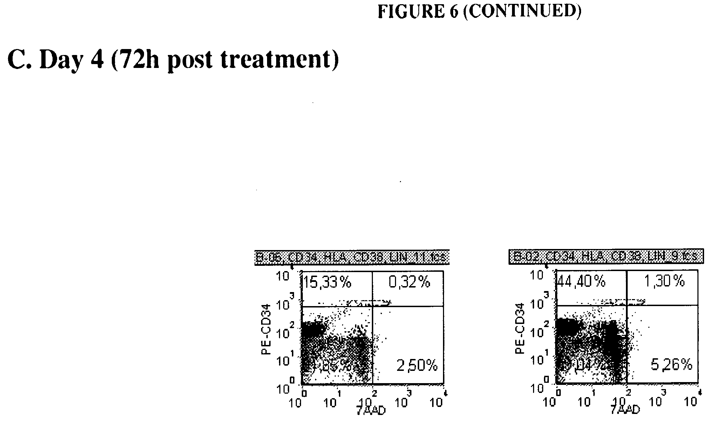

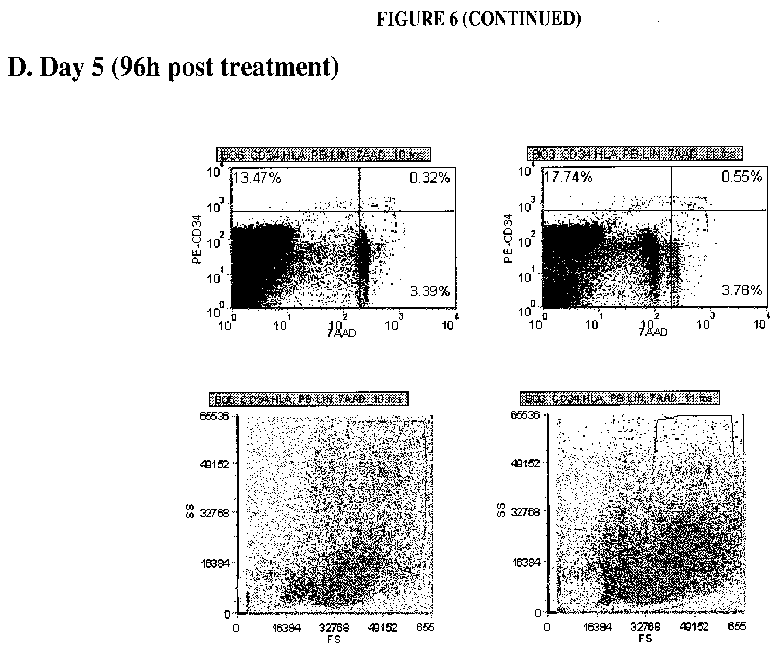

Subpopulations of total white blood cells (WBC) from the BM and PB were analyzed by FACS to select hematopoietic pluripotent stem cells presenting CD34⁺ (Lin⁻CD34⁺CD38⁻) markers, which is the marker commonly used in clinical setting to purify HSC from either PB or BM (See Figure 6). Shaded cells in Table 5 indicate the level of CD34⁺ cells expressed in the BM. The non-highlighted cells in Table 5 indicate the number of CD34⁺ cells present in the PB. Table 7 shows that lipopeptides can stimulate CD34⁺ proliferation and mobilization from the BM to the PB. Table 7 also indicates that even a single BM draw can stimulate CD34⁺ cell proliferation and mobilization in the PB. These data are further summarized in Figure 5. Figure 5 shows that the level of CD34+ cells recruited to the PB peaked after treatment with CBLB612.

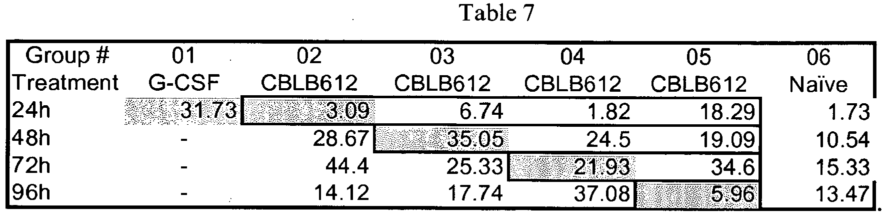

### Example 6

### HSC Mobilization is CBLB612 Dose-Dependent

This was demonstrated by administering adjusted doses of drugs proportional to the average weight of mice in a given distribution group. The doses were calculated as intended dose/kg of average weight. The adjustment of a dose was accomplished by adjusting the concentration of an article for injection while keeping the injection volume constant. See Table 8.

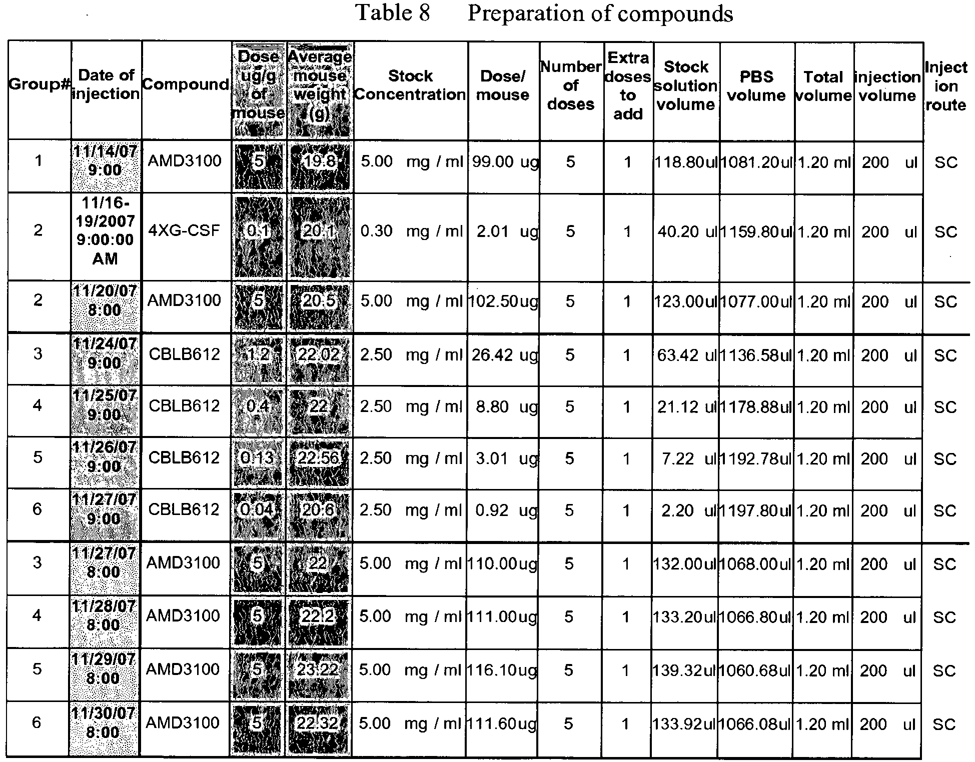

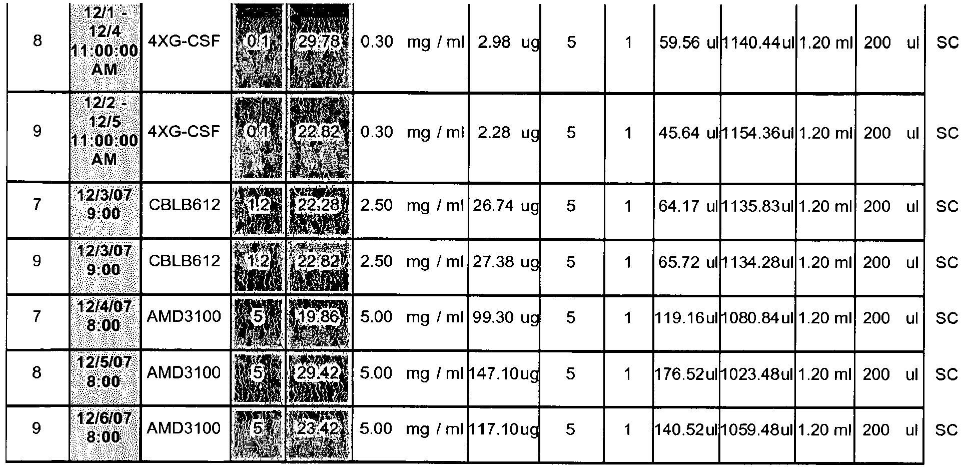

According to Table 9, all groups received a single subcutaneous (SC) injection of 5 mg/kg of AMD3100 one hour before euthanasia. Control group 1 was injected with 5 mg/kg of AMD3100 only. Control group 2 was treated with four daily 0.1 mg/kg SC injections of G-CSF and on the fifth day the mice were injected with AMD3100. Control group 8 consisted of ICR mice and received the same treatment as Control group 2. Study groups 3, 4, 5, and 6 received a single SC injection of CBLB612 at 1.2; 0.4; 0.13; and 0.04 mg/kg doses, respectively, at 72 hours before blood collection. Group 7 received a single SC injection of 1.2 mg/kg CBLB612 at 24 hours before blood collection. Study group 9 was treated with four daily SC injections of 0.1 mg/kg G-CSF, a single SC injection of 1.2 mg/kg CBLB612 at 72 hours before blood collection, and a single SC injection of 5 mg/kg AMD3100 one hour before euthanasia at the fifth day after the first G-CSF injection.

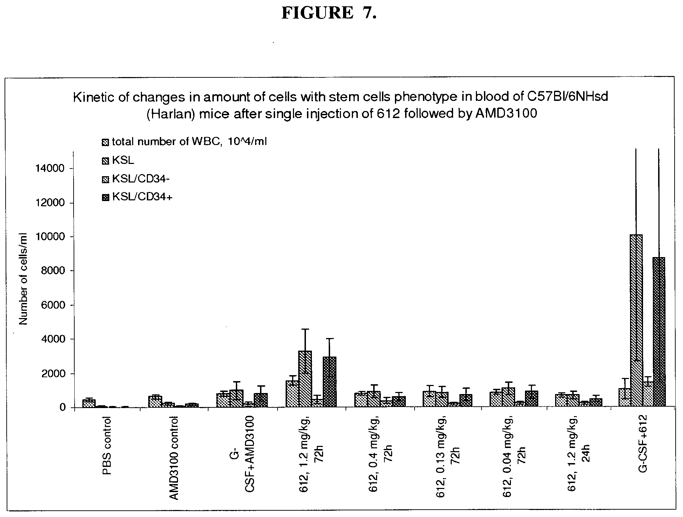

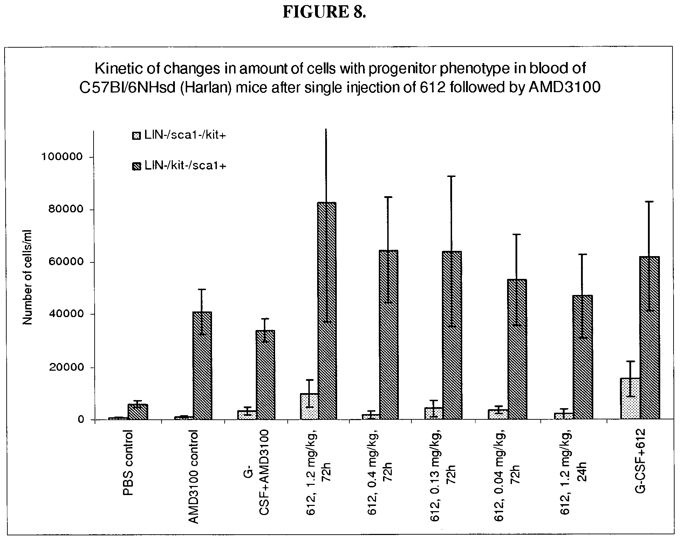

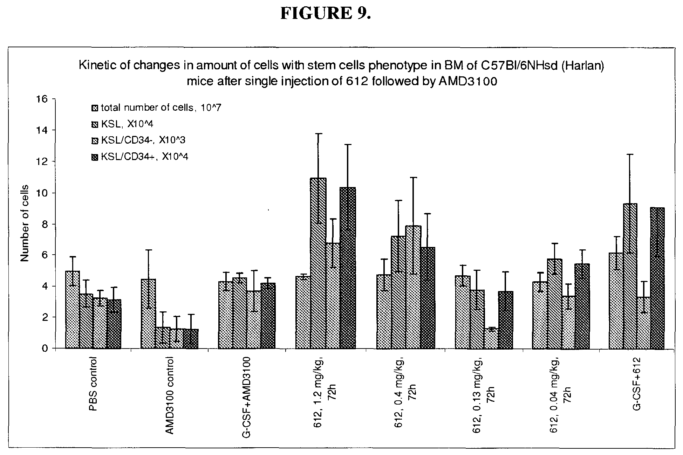

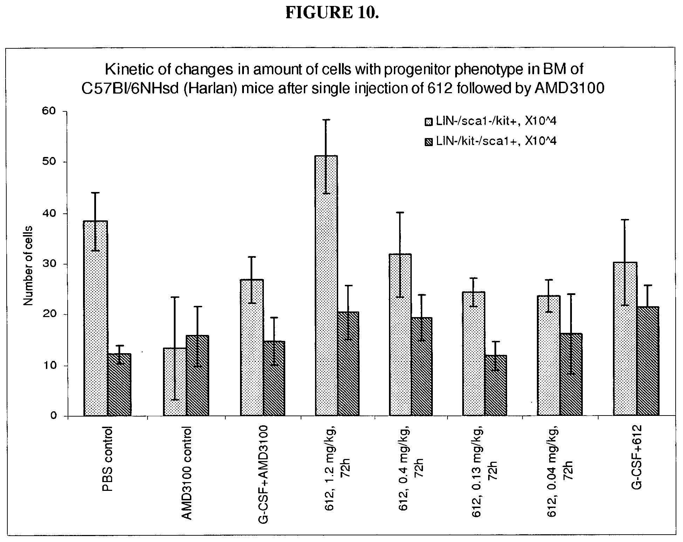

The measured numbers of hematopoietic stem and progenitor cells in the peripheral blood of C57B1 mice co-treated with a single injection of different doses of CBLB612 followed by a single injection of AMD3100 one hour before blood collection revealed that the minimum dose of CBLB612 that leads to maximum HSC and progenitor cell mobilization is 1.2 mg/kg. See Table 10 and Figures 7-8. This dose also lead to a maximum increase of HSC in bone marrow. See Table 11 and Figures 9-10. Treatment of C57B1/6NHsd mice with CBLB612 plus AMD3100 was approximately 3 times more efficient for HSC mobilization than treatment with G-CSF plus AMD3100, while treatment of those mice with all three compounds mobilized approximately twice more HSC than combinations of CBLB612 plus AMD3100; and G-CSF plus AMD3100 together. See Table 10. ICR mice showed higher efficiency of mobilization with G-CSF plus AMD3100 than did C57B1/6NHsd mice. See Table 10.

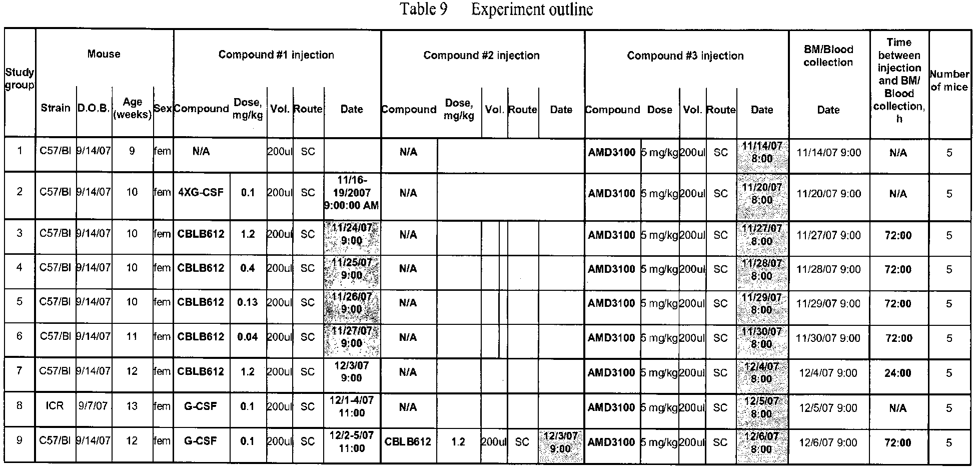

**Table 10 Average numbers and standard deviation of different hematopoietic stem and progenitor cells populations in peripheral blood of C57B1 mice.**

| **AVERAGE** | **9-11 weeks old C57BI/6NHsd mice from Harlan** | | | | | | |
|---|---|---|---|---|---|---|---|
| | **total number of WBC, 10^4/ml** | **7AAD-/LIN-** | **LIN-/sca1- /kit+** | **LIN-/kit- /sca1 +** | **KSL** | **KSL/ CD34-** | **KSL/C D34+** |
| PBS control | 440.06 | 215328 | 545 | 5843 | 51 | 24 | 20 |
| **AMD3100 control** | **629.95** | **254725** | **1104** | **40971** | **238** | **72** | **174** |
| **G-CSF+AMD3100** | **790.37** | **354183** | **3215** | **33927** | **967** | **186** | **800** |
| **612, 1.2 mg/kg, 72h** | **1552.69** | **883328** | **9919** | **82532** | **3262** | **447** | **2898** |
| 612, 0.4 mg/kg, 72h | 805.90 | 412675 | 1808 | 64526 | 913 | 356 | 606 |
| 612, 0.13 mg/kg, 72h | 908.56 | 530552 | 4262 | 64057 | 849 | 177 | 711 |
| 612, 0.04 mg/kg, 72h | 860.27 | 439053 | 3508 | 53310 | 1079 | 228 | 869 |
| **612, 1.2 mg/kg, 24h** | **670.01** | **302148** | **1991** | **47141** | **670** | **226** | **467** |
| **ICR, G-CSF+AMD** | **1713.21** | **364145** | **14937** | **39941** | **10193** | **434** | **9824** |
| **G-CSF+612** | **1048.60** | **628653** | **15394** | **61946** | **10072** | **1459** | **8689** |
| | | | | | | | |

| **STDEV** | | | | | | | |
|---|---|---|---|---|---|---|---|
| PBS control | 89.18 | 83287 | 528 | 1263 | 39 | 20 | 15 |
| AMD3100 control | 119.85 | 42288 | 481 | 8492 | 81 | 51 | 62 |
| G-CSF+AMD3100 | 146.76 | 126834 | 1318 | 4318 | 538 | 133 | 436 |
| 612, 1.2 mg/kg, 72h | 284.07 | 212955 | 5213 | 45391 | 1278 | 225 | 1119 |
| 612, 0.4 mg/kg, 72h | 91.29 | 152849 | 1491 | 20025 | 365 | 168 | 257 |
| 612, 0.13 mg/kg, 72h | 336.08 | 211661 | 3077 | 28548 | 315 | 92 | 382 |
| 612, 0.04 mg/kg, 72h | 112.75 | 70877 | 1428 | 17371 | 367 | 62 | 354 |
| 612, 1.2 mg/kg, 24h | 146.07 | 92627 | 1828 | 15919 | 206 | 65 | 156 |
| ICR, G-CSF+AMD | 580.45 | 100699 | 6563 | 20748 | 7393 | 292 | 7250 |
| G-CSF+612 | 119.39 | 73673 | 2812 | 17783 | 3660 | 112 | 3585 |

**Table 11 Average numbers and standard deviation of different hematopoietic stem and progenitor cells populations in mouse bone marrow.**

| **AVERAGE** | | | | | | | |
|---|---|---|---|---|---|---|---|
| | **total number of cells, 10^7** | **7AAD-/LIN-, X10^6** | **LIN-/sca1- /kit+, X10^4** | **LIN-/kit- /sca1+, X10^4** | **KSL, X10^4** | **KSL/CD34-, X10^3** | **KSL/CD34+ X10^4** |
| PBS control | 4.96 | 2.55 | 38.35 | 12.14 | 3.53 | 3.28 | 3.16 |
| AMD3100 control | 4.47 | 2.39 | 13.26 | 15.74 | 1.37 | 1.24 | 1.25 |
| G-CSF+AMD3100 | 4.32 | 2.08 | 26.75 | 14.60 | 4.56 | 3.70 | 4.21 |
| 612, 1.2 mg/kg, 72h | 4.65 | 2.27 | 51.15 | 20.39 | 10.93 | 6.77 | 10.35 |
| 612, 0.4 mg/kg, 72h | 4.77 | 2.20 | 31.84 | 19.32 | 7.23 | 7.89 | 6.54 |
| 612, 0.13 mg/kg, 72h | 4.72 | 1.95 | 24.33 | 11.85 | 3.80 | 1.28 | 3.71 |
| 612, 0.04 mg/kg, 72h | 4.32 | 1.97 | 23.63 | 16.19 | 5.78 | 3.42 | 5.48 |
| G-CSF+612 | 6.19 | 1.62 | 30.14 | 21.46 | 9.32 | 3.37 | 9.09 |

| **STDEV** | | | | | | | |
|---|---|---|---|---|---|---|---|
| PBS control | 0.92 | 0.46 | 5.69 | 1.82 | 0.88 | 0.50 | 0.81 |
| AMD3100 control | 1.86 | 0.78 | 10.16 | 5.91 | 1.01 | 0.80 | 0.94 |
| G-CSF+AMD3100 | 0.58 | 0.43 | 4.65 | 4.72 | 0.32 | 1.30 | 0.36 |
| 612, 1.2 mg/kg, 72h | 0.19 | 0.14 | 7.23 | 5.33 | 2.86 | 1.54 | 2.75 |
| 612, 0.4 mg/kg, 72h | 0.98 | 0.35 | 8.34 | 4.53 | 2.29 | 3.08 | 2.11 |
| 612, 0.13 mg/kg, 72h | 0.64 | 0.23 | 2.83 | 2.86 | 1.25 | 0.13 | 1.26 |
| 612, 0.04 mg/kg, 72h | 0.61 | 0.44 | 3.24 | 7.89 | 0.97 | 0.79 | 0.90 |
| G-CSF+612 | 1.06 | 0.39 | 8.40 | 4.29 | 3.15 | 0.99 | 3.11 |

After treatment of the mice with: a single dose of 1.2 mg/kg CBLB612 72 hours before blood collection, plus four daily injections of 0.1 mg/kg G-CSF and a single injection of 5 mg/kg AMD3100 one hour before euthanasia, mobilization of HSC was found to be synergistic (approximately 6-8 times higher) as compared to treatment with two drugs: CBLB612 and AMD3100 or G-CSF with AMD1300. The minimum dose that will lead to a maximum increase of HSC in bone marrow was found to be 1.2 mg/kg CBLB612.

### Example 7

### Kinetics of HSC Mobilization

The following mobilization of HSC and progenitor cells from the bone marrow to peripheral blood after CBLB612 injection. This was demonstrated by administering adjusted doses of drugs proportional to the average weight of mice in a given distribution group. The doses were calculated as intended dose/kg of average weight. The adjustment of a dose was accomplished by adjusting the concentration of an article for injection while keeping the injection volume constant. See Table 12.

According to Table 13, with the exception of the PBS control group 8, all groups received a 5 mg/kg dose of AMD3100 one hour before euthanasia. Control group 7 received a 6.5 mg/kg does of AMD3100. Negative control group 8 received a single SC injection of 200 µl PBS one hour before blood collection. Study groups 1-6 received single SC injections of CBLB12 at 2 mg/kg at different time points prior to blood collection. Positive control group 9 received four daily SC injections of 0.1 mg/kg G-CSF.

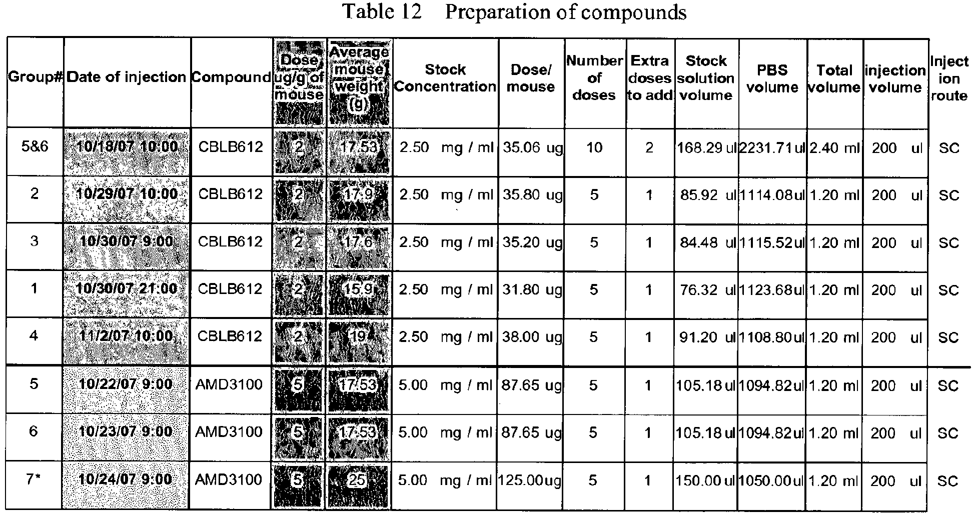

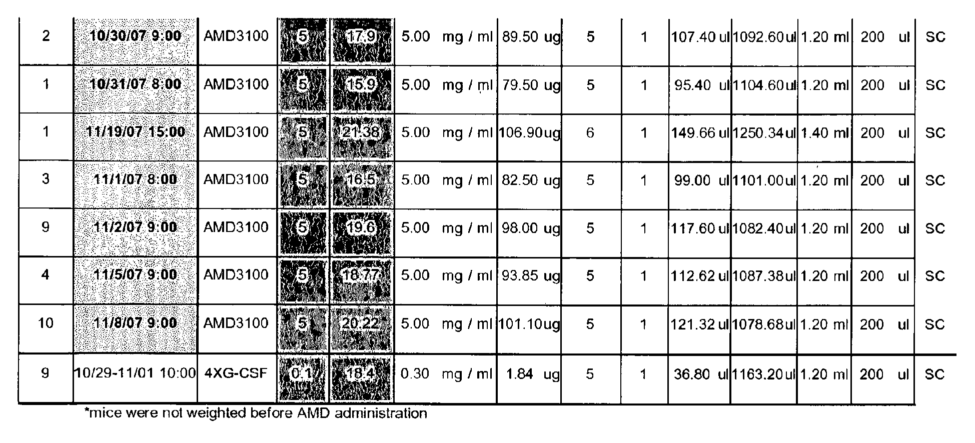

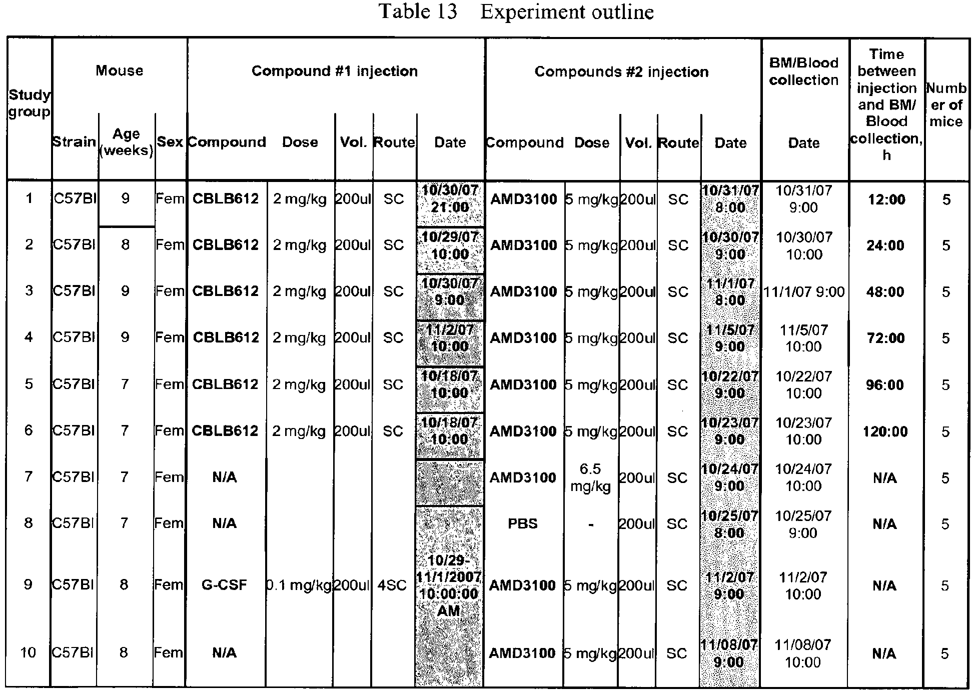

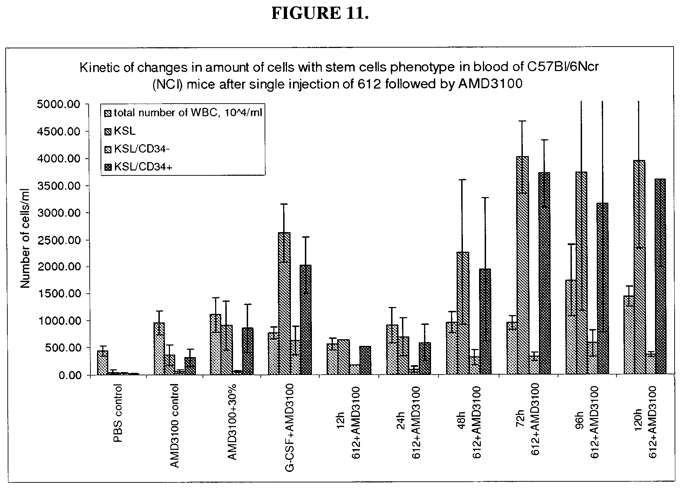

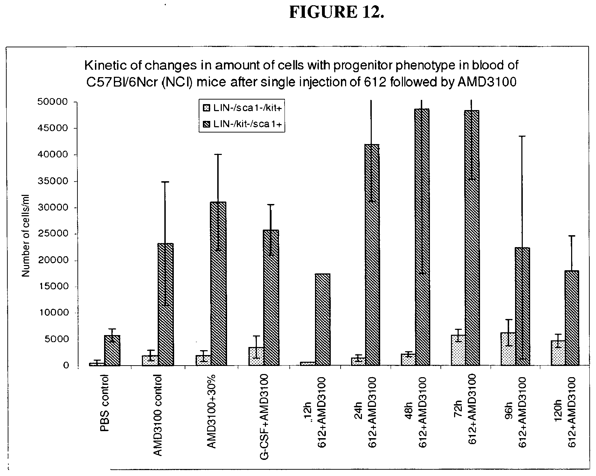

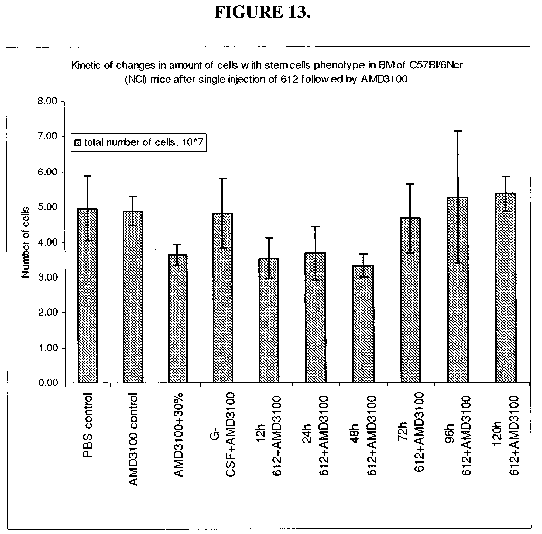

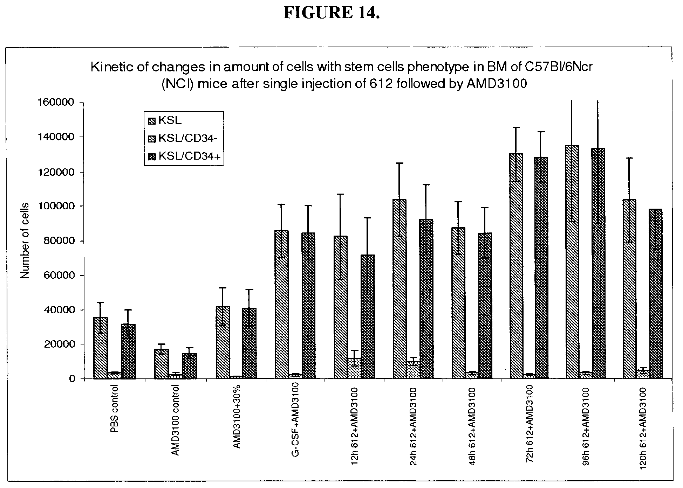

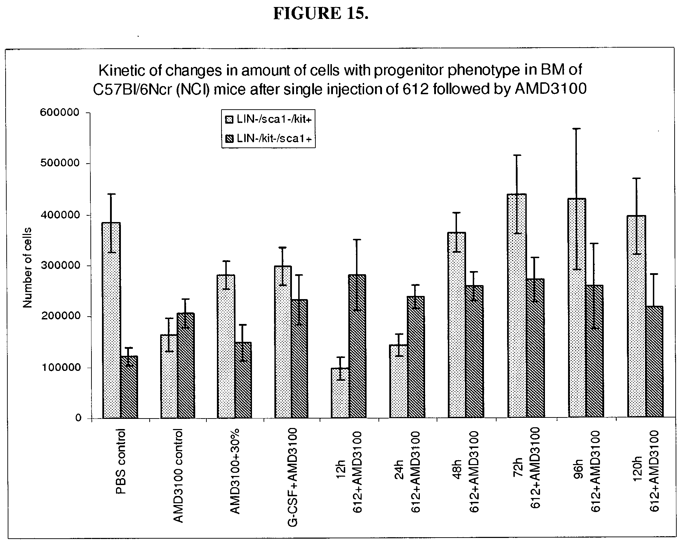

Measured numbers of hematopoietic stem and progenitor cells in the peripheral blood of C57B1 mice co-treated with a single injection of CBLB612 at different time points followed by a single injection of AMD3100 one hour before blood collection revealed that the maximum mobilization of these cells occurs around 72-96 hours after CBLB612 injection. True pluripotent HSC (KSL/CD34⁻), ST-HSC (KSL/CD34⁻) and committed progenitors (most committed to myeloid lineage; LIN⁻/sca1⁻/kit⁺) were all increased. See Table 14 and Figures 11-12. Single injection of CBLB612 increased numbers of HSC in bone marrow with the peak around 12-24 hours after injection. See Table 15 and Figures 13-15.

**Table 14 Average numbers and standard deviation of different hematopoietic stem and progenitor cells populations in peripheral blood of C57B1 mice.**

| **AVERAGE** | | | | | | | | |
|---|---|---|---|---|---|---|---|---|
| | **total number of WBC, 10^4/ml** | **7AAD-/LIN-** | **LIN-/sca1-/kit+** | **LIN-/kit- /sca1+** | **KSL** | **KSL/CD34-** | **KSL/CD34+** | |
| PBS control | 440.06 | 215328 | 545 | 5843 | 51 | 24 | 20 | |
| AMD3100 control | 962.40 | 421616 | 1932 | 23142 | 360 | 56 | 308 | |
| AMD3100+30% | 1111.05 | 302644 | 1816 | 30938 | 907 | 63 | 856 | |
| G-CSF+AMD3100 | 763.05 | 192186 | 3493 | 25679 | 2615 | 625 | 2015 | |
| 12h 612+AMD3100 | 558.81 | 192528 | 647 | 17295 | 649 | 175 | 512 | one mouse for KSL |
| 24h 612+AMD3100 | 908.56 | 216045 | 1392 | 41923 | 693 | 100 | 587 | |
| 48h 612+AMD3100 | 963.11 | 228528 | 2173 | 48581 | 2250 | 313 | 1941 | |
| 72h 612+AMD3100 | 954.86 | 325024 | 5748 | 48271 | 4006 | 328 | 3710 | |
| 96h 612+AMD3100 | 1741.67 | 269560 | 6212 | 22392 | 3733 | 574 | 3151 | |
| 120h 612+AMD3100 | 1442.19 | 121125 | 4683 | 17996 | 3930 | 364 | 3592 | |
| | | | | | | | | |

| **STDEV** | | | | | | | | |
|---|---|---|---|---|---|---|---|---|
| PBS control | 89.18 | 83287 | 527.5 | 1263 | 38.9 | 19.536 | 14.766 | |
| AMD3100 control | 219.04 | 118188 | 965.1 | 11701 | 184 | 32.622 | 159.16 | |
| AMD3100+30% | 322.48 | 142796 | 1039 | 9048 | 452 | 19.682 | 452.32 | |
| G-CSF+AMD3100 | 109.98 | 75046 | 2089 | 4813 | 533 | 263.54 | 517.66 | |
| 12h 612+AMD3100 | 108.12 | | | | | | | |
| 24h 612+AMD3100 | 336.08 | 50984 | 655.1 | 10826 | 355 | 56.838 | 336.83 | |
| 48h 612+AMD3100 | 190.76 | 76429 | 462.3 | 31068 | 1333 | 145.5 | 1325.4 | |
| 72h 612+AMD3100 | 129.58 | 37064 | 1178 | 13005 | 664 | 84.876 | 622.28 | |
| 96h 612+AMD3100 | 655.51 | 123978 | 2533 | 21114 | 2554 | 244.52 | 2368.1 | |
| 120h 612+AMD3100 | 195.08 | 31048 | 1273 | 6481 | 1591 | 45.778 | 1594.6 | |

**Table 15 Average numbers and standard deviation of different hematopoietic stem and progenitor cells populations in bone marrow of C57B1 mice.**

| **AVERAGE** | | | | | | | |
|---|---|---|---|---|---|---|---|
| | **total number cells, 10^7** | **of 7AAD-/LIN-**, **X10^6** | **LIN-/sca1- /kit+** | **LIN-/kit-/sca1+** | **KSL** | **KSL/CD34-** | **KSL/CD34+** |
| PBS control | 4.96 | 2.55 | 383498 | 121377 | 35315 | 3278 | 31613 |
| AMD3100 control | 4.88 | 2.44 | 163895 | 206061 | 17184 | 2689 | 14743 |
| AMD3100+30% | 3.65 | 2.26 | 281722 | 148328 | 41791 | 1084 | 40873 |
| G-CSF+AMD3100 | 4.81 | 2.73 | 298469 | 232597 | 85717 | 2256 | 84436 |
| 12h 612+AMD3100 | 3.54 | 3.82 | 97504 | 281015 | 82228 | 11752 | 71428 |
| 24h 612+AMD3100 | 3.69 | 3.35 | 143400 | 237956 | 103700 | 9930 | 92231 |
| 48h 612+AMD3100 | 3.33 | 2.39 | 364059 | 259442 | 87164 | 3519 | 84644 |
| 72h 612+AMD3100 | 4.67 | 2.24 | 438649 | 271954 | 129830 | 2417 | 128131 |
| 96h 612+AMD3100 | 5.27 | 2.38 | 428666 | 258125 | 134957 | 3623 | 132801 |
| 120h 612+AMD3100 | 5.37 | 2.29 | 394716 | 217962 | 103493 | 4720 | 98345 |
| | | | | | | | |

| **STDEV** | | | | | | | |
|---|---|---|---|---|---|---|---|
| PBS control | 0.92 | 0.46 | 56888 | 18160 | 8765 | 497 | 8109 |
| AMD3100 control | 0.42 | 0.43 | 32571 | 28608 | 3029 | 896 | 3193 |
| AMD3100+30% | 0.30 | 0.39 | 28015 | 35207 | 10763 | 272 | 10537 |
| G-CSF+AMD3100 | 0.99 | 0.32 | 37404 | 48926 | 15449 | 552 | 15572 |
| 12h 612+AMD3100 | 0.58 | 0.85 | 22158 | 69727 | 24942 | 4260 | 21930 |
| 24h 612+AMD3100 | 0.76 | 0.59 | 20687 | 22863 | 21014 | 2119 | 20246 |
| 48h 612+AMD3100 | 0.32 | 0.20 | 38395 | 27906 | 15188 | 912 | 14523 |
| 72h 612+AMD3100 | 0.97 | 0.36 | 76670 | 42390 | 15423 | 540 | 14793 |
| 96h 612+AMD3100 | 1.88 | 0.93 | 138460 | 83849 | 43578 | 1039 | 43032 |
| 120h 612+AMD3100 | 0.50 | 0.63 | 74236 | 62739 | 24257 | 1621 | 23833 |

Single injection of CBLB612 increases numbers of HSC in bone marrow with a peak of around 12-24 hours after injection.

## Claims

1. A composition for use in treating a disease in a mammal, the composition comprising a lipopeptide, wherein the lipopeptide is a compound of the formula: 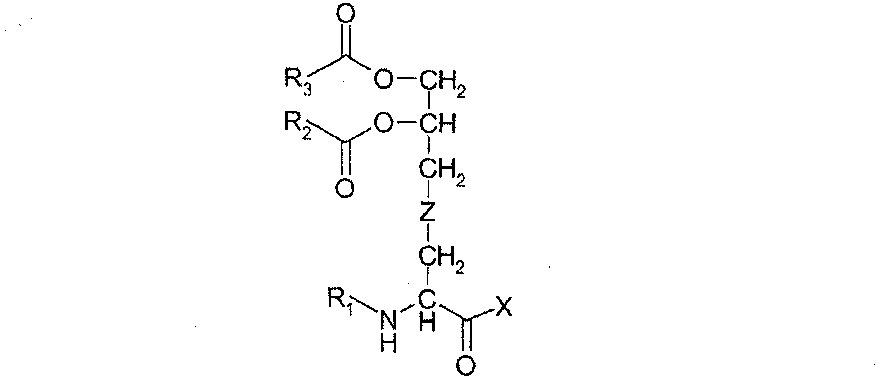 wherein,
R₁ represents H or -CO-R₄,
R₂, R₃ and R₄ independently are H or optionally substituted C₈-C₁₆ aliphatic;
X is a peptide selected from the group consisting of SEQ ID NOs: 8, 16-18, 20, and 21;
Z is S or CH₂ and
the disease is selected from the group consisting of cancer, an autoimmune disease, an anemia, a non-malignant hematological disorder, Wiskott-Aldrich syndrome, Chediak-Higashi syndrome, Severe Combined Immune Deficiency (SCID), thalassemia, and sickle cell anemia.

2. The composition for use of claim 1, wherein the cancer is selected from the group consisting of leukemia, lymphoma, a malignant hematological disorder, breast cancer, testicular cancer, neuroblastoma, ovarian cancer, myeloma, and Waldenstrom's Macroglobulinemia

3. The composition for use according to claim 1-2, wherein R₁ is H and R₂ and R₃ are C₁₆ aliphatics or substitutions thereof.

4. The composition for use according to claim 1-2, wherein the compound is an RR or RS stereoisomer, or mixture thereof.

5. The composition for use according to claim 1-2, wherein further a radioprotectant is to be administered.

6. The composition for use according to claim 5, wherein the radioprotectant is an antioxidant.

7. The composition for use according to claim 5, wherein the radioprotectant is a cytokine.

8. The composition for use according to claim 7, wherein the cytokine is a stem cell factor.

9. The composition for use according to claim 5, wherein the radioprotectant is flagellin.

10. The composition for use according to any one of claims 1-9, wherein the mobility of hematopoietic stem cells is increased.

11. The composition for use according to claim 10, wherein the mobility of the hematopoietic stem cells increases from the bone marrow to the blood stream.

## Patentansprüche

1. Zusammensetzung zur Verwendung in der Behandlung einer Erkrankung bei einem Säuger, wobei die Zusammensetzung ein Lipopeptid umfasst, wobei das Lipopeptid eine Verbindung mit der folgenden Formel ist: 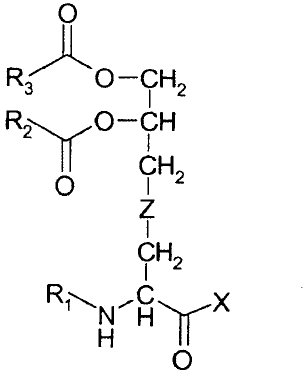 wobei
R₁ für H oder -CO-R₄ steht,
R₂, R₃ und R₄ jeweils unabhängig H und gegebenenfalls substituierter C₈-C₁₆-aliphatischer Rest sind;
X ein Peptid ausgewählt aus der Gruppe bestehend aus SEQ ID NOs: 8, 16-18, 20 und 21 ist;
Z S oder CH₂ ist, und
die Erkrankung ausgewählt ist aus der Gruppe bestehend aus Krebs, einer Autoimmunerkrankung, einer Anämie, einer nicht-malignen hämatologischen Erkrankung, Wiskott-Aldrich-Syndrom, Chediak-Higashi-Syndrom, schwerem kombinierten Immundefekt (SCID), Thalassämie und Sichelzellanämie.

2. Zusammensetzung zur Verwendung nach Anspruch 1, wobei der Krebs ausgewählt ist aus der Gruppe bestehend aus Leukämie, Lymphom, einer malignen hämatologischen Erkrankung, Brustkrebs, Hodenkrebs, Neuroblastom, Ovarialkrebs, Myelom und Waldenström'scher Makroglobulinämie.

3. Zusammensetzung zur Verwendung nach Anspruch 1-2, wobei R₁ H ist und R₂ und R₃ C₁₆-aliphatische Reste oder substituierte Formen davon sind.

4. Zusammensetzung zur Verwendung nach Anspruch 1-2, wobei die Verbindung ein RR- oder RS-Stereoisomer oder eine Mischung davon ist.

5. Zusammensetzung zur Verwendung nach Anspruch 1-2, wobei ferner ein Strahlenschutzmittel zu verabreichen ist.

6. Zusammensetzung zur Verwendung nach Anspruch 5, wobei das Strahlenschutzmittel ein Antioxidans ist.

7. Zusammensetzung zur Verwendung nach Anspruch 5, wobei das Strahlenschutzmittel ein Zytokin ist.

8. Zusammensetzung zur Verwendung nach Anspruch 7, wobei das Zytokin ein Stammzellfaktor ist.

9. Zusammensetzung zur Verwendung nach Anspruch 5, wobei das Strahlenschutzmittel Flagellin ist.

10. Zusammensetzung zur Verwendung nach einem der Ansprüche 1-9, wobei die Mobilität hämatopoietischer Stammzellen erhöht wird.

11. Zusammensetzung zur Verwendung nach Anspruch 10, wobei die Mobilität der hämatopoietischen Stammzellen vom Knochenmark zum Blutstrom erhöht wird.

## Revendications

1. Composition pour une utilisation dans le traitement d'une maladie chez un mammifère, la composition comprenant un lipopeptide, dans laquelle le lipopeptide est un composé de la formule : 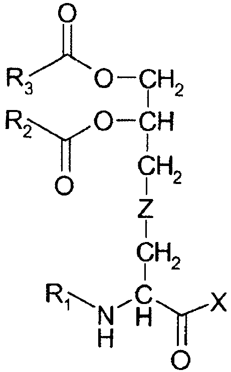 dans laquelle,
R₁ représente H ou -CO-R₄,
R₂, R₃ et R₄ sont indépendamment H ou un groupe aliphatique en C₈-C₁₆ éventuellement substitué ;
X est un peptide choisi dans le groupe comprenant les SEQ ID NO : 8, 16 à 18, 20, et 21 ;
Z est S ou CH₂ et
la maladie est choisie dans le groupe comprenant le cancer, une maladie auto-immune, une anémie, une pathologie hématologique non maligne, le syndrome de Wiskott-Aldrich, le syndrome de Chediak-Higashi, un déficit immunitaire combiné sévère (SCID), une thalassémie et une anémie à hématies falciformes.

2. Composition pour une utilisation selon la revendication 1, dans laquelle le cancer est choisi dans le groupe comprenant la leucémie, le lymphome, une pathologie hématologique maligne, le cancer du sein, le cancer du testicule, un neuroblastome, le cancer de l'ovaire, un myélome et la macroglobulinémie de Waldenstrom.

3. Composition pour une utilisation selon les revendications 1 et 2, dans laquelle R₁ est H et R₂ et R₃ sont des groupes aliphatiques en C₁₆ ou des substitutions de ceux-ci.

4. Composition pour une utilisation selon les revendications 1 et 2, dans laquelle le composé est un stéréo-isomère RR ou RS, ou un mélange de ceux-ci.

5. Composition pour une utilisation selon les revendications 1 et 2, dans laquelle en outre un radioprotecteur doit être administré.

6. Composition pour une utilisation selon la revendication 5, dans laquelle le radioprotecteur est un antioxydant.

7. Composition pour une utilisation selon la revendication 5, dans laquelle le radioprotecteur est une cytokine.

8. Composition pour une utilisation selon la revendication 7, dans laquelle la cytokine est un facteur de cellules souches.

9. Composition pour une utilisation selon la revendication 5, dans laquelle le radioprotecteur est la flagelline.

10. Composition pour une utilisation selon l'une quelconque des revendications 1 à 9, dans laquelle la mobilité des cellules souches hématopoïétiques est accrue.

11. Composition pour une utilisation selon la revendication 10, dans laquelle la mobilité des cellules souches hématopoïétiques augmente depuis la moelle osseuse jusque dans le flux sanguin.


### SEQUENCE LISTING

<110> Clevel and Biol abs, Inc. Shakhov, Alexander N. Strom Evgueni a
<120> Methods for Increasing and Mobilizing Hematopoietic Stem Cells
<130> 050991.0401.04PC00
<150> US 60/884, 162
   <151> 2007-01-09
<150> US 60/889, 893
   <151> 2007-02-14
<150> US 60/938, 564
   <151> 2007-05-17
<150> US 61/013, 243
   <151> 2007- 12- 12
<160> 52
<170> PatentIn version 3.3
<210> 1
   <211> 4
   <212> PRT
   <213> Artificial
<220>
   <223> SYNTHETIC PEPTIDE
<400> 1
   Ser Asn Asn Ala
   1
<210> 2
   <211> 5
   <212> PRT
   <213> Artificial
<220>
   <223> SYNTHETIC PEPTI DE
<400> 2
   Gly Ser Ser His His
   1               5
<210> 3
   <211> 5
   <212> PRT
   <213> Artificial
<220>
   <223> SYNTHETIC PEPTI DE
<400> 3
   Lys Gln Asn Val Ser
   1               5
<210> 4
   <211> 5
   <212> PRT
   <213> Artificial
<220>
   <223> SYNTHETIC PEPTIDE
<400> 4
   Asn Asn Ser Gly Lys
   1               5
<210> 5
   <211> 5
   <212> PRT
   <213> Artificial
<220>
   <223> SYNTHETIC PEPTI DE
<400> 5
   Gln Pro Asp Arg Tyr
   1               5
<210> 6
   <211> 5
   <212> PRT
   <213> Artificial
<220>
   <223> SYNTHETIC PEPTI DE
<400> 6
   Arg Pro Asp Arg Tyr
   1               5
<210> 7
   <211> 5
   <212> PRT
   <213> Artificial
<220>
   <223> SYNTHETIC PEPTI DE
<400> 7
   Ser Glu Glu Glu Glu
   1 5
<210> 8
   <211> 5
   <212> PRT
   <213> Artificial
<220>
   <223> SYNTHETIC PEPTI DE
<400> 8
   Ser Lys Lys Lys Lys
   1 5
<210> 9
   <211> 5
   <212> PRT
   <213> Artificial
<220>
   <223> SYNTHETIC PEPTI DE
<400> 9
   Ser Asn Asn Asn Ala
   1 5
<210> 10
   <211> 5
   <212> PRT
   <213> Artificial
<220>
   <223> SYNTHETIC PEPTI DE
<400> 10
   Ser Pro Pro Pro Pro
   1 5
<210> 11
   <211> 5
   <212> PRT
   <213> Artificial
<220>
   <223> SYNTHETIC PEPTI DE
<400> 11
   Gly Gln His His Met
   1 5
<210> 12
   <211> 5
   <212> PRT
   <213> Artificial
<220>
   <223> SYNTHETIC PEPTI DE
<400> 12
   Gly Gln His His His
   1               5
<210> 13
   <211> 5
   <212> PRT
   <213> Artificial
<220>
   <223> SYNTHETIC PEPTI DE
<400> 13
   Ser Ser His His Met
   1               5
<210> 14
   <211> 5
   <212> PRT
   <213> Artificial
<220>
   <223> SYNTHETIC PEPTI DE
<400> 14
   Gly Ser His His Met
   1               5
<210> 15
   <211> 5
   <212> PRT
   <213> Artificial
<220>
   <223> SYNTHETIC PEPTIDE
<400> 15
   Ser Gln Met His His
   1               5
<210> 16
   <211> 5
   <212> PRT
   <213> Artificial
<220>
   <223> SYNTHETIC PEPTI DE
<400> 16
   Gly Glu Thr Asp Lys
   1 5
<210> 17
   <211> 5
   <212> PRT
   <213> Artificial
<220>
   <223> SYNTHETIC PEPTIDE
<400> 17
   Gly Glu Glu Ser Asn
   1 5
<210> 18
   <211> 5
   <212> PRT
   <213> Artificial
<220>
   <223> SYNETHI C PEPTI DE
<400> 18
   Gly Glu Glu Asp Asp
   1 5
<210> 19
   <211> 6
   <212> PRT
   <213> Artificial
<220>
   <223> SYNTHETIC PEPTIDE
<400> 19
   Thr Glu Asn Val Lys Glu
   1 5
<210> 20
   <211> 8
   <212> PRT
   <213> Artificial
<220>
   <223> SYNTHETIC PEPTIDE
<400> 20
   Gln Gly Glu Glu Ser Asn Asp Lys
   1 5
<210> 21
   <211> 9
   <212> PRT
   <213> Artificial
<220>
   <223> SYNTHETIC PEPTI DE
<400> 21
   Val Gln Gly Glu Glu Ser Asn Asp Lys
   1 5
<210> 22
   <211> 9
   <212> PRT
   <213> Artificial
<220>
   <223> SYNTHETIC PEPTI DE
<400> 22
   Phe Glu Pro Pro Pro Ala Thr Thr Thr
   1 5
<210> 23
   <211> 9
   <212> PRT
   <213> Art i f i ci al
<220>
   <223> SYNTHETIC PEPTI DE
<400> 23
   Gly Asp Lys Tyr Phe Lys Glu Thr Glu
   1 5
<210> 24
   <211> 9
   <212> PRT
   <213> Artificial
<220>
   <223> SYNTHETIC PEPTI DE
<400> 24
   Gly Asp Pro Lys His Pro Lys Ser Phe
   1 5
<210> 25
   <211> 9
   <212> PRT
   <213> Artificial
<220>
   <223> SYNTHETIC PEPTI DE
<400> 25
   Gly Gly Gln Glu Lys Ser Ala Ala Gly
   1 5
<210> 26
   <211> 9
   <212> PRT
   <213> Artificial
<220>
   <223> SYTHETIC PEPTI DE
<400> 26
   Gly Pro Cys Pro Gly Cys Pro Pro Cys
   1 5
<210> 27
   <211> 9
   <212> PRT
   <213> Artificial
<220>
   <223> SYNTHETIC PEPTIDE
<400> 27
   Pro Pro Cys Pro Gly Cys Pro Pro Cys
   1 5
<210> 28
   <211> 11
   <212> PRT
   <213> Artificial
<220>
   <223> SYNTHETIC PEPTI DE
<400> 28
   Asp Asn Glu Glu Lys Pro Thr Pro Glu Gln Asp
   1 5 10
<210> 29
   <211> 11
   <212> PRT
   <213> Artificial
<220>
   <223> SYNTHETIC PEPTI DE
<400> 29
   Gly Asn Gly Gly Ala Pro Ala Gln Pro Lys Gly
   1 5 10
<210> 30
   <211> 12
   <212> PRT
   <213> Artificial
<220>
   <223> SYNTHETIC PEPTI DE
<400> 30
   Phe Glu Pro Pro Pro Ala Thr Thr Thr Lys Ser Lys
   1               5                   10
<210> 31
   <211> 13
   <212> PRT
   <213> Artificial
<220>
   <223> SYNTHETIC PEPTI DE
<400> 31
   Gly Asn Asn Asp Glu Ser Asn Ile Ser Phe Lys Glu Lys
   1               5                   10
<210> 32
   <211 > 14
   <212> PRT
   <213> Artificial
<220>
   <223> SYNTHETIC PEPTI DE
<400> 32
   Gly Asp Pro Lys His Pro Lys Ser Phe Thr Gly Trp Val Ala
   1               5                   10
<210> 33
   <211> 15
   <212> PRT
   <213> Artificial
<220>
   <223> SYNTHETIC PEPTI DE
<400> 33
<210> 34
   <211> 15
   <212> PRT
   <213> Artificial
<220>
   <223> SYNTHETIC PEPTI DE
<400> 34
   Asn Lys Asp Asn Glu Ala Glu Pro Val Thr Glu Gly Asn Ala Thr
   1 5 10 15
<210> 35
   <211> 15
   <212> PRT
   <213> Artificial
<220>
   <223> SYNTHETIC PEPTI DE
<400> 35
   Ser Lys Glu Gly Asn Gly Pro Asp Pro Asp Asn Ala Ala Lys Ser
   1 5 10 15
<210> 36
   <211> 16
   <212> PRT
   <213> Artificial
<220>
   <223> SYNTHETIC PEPTI DE
<400> 36
   Gly Asp Lys Thr Pro Ser Thr Lys Ser Ala Gly Lys Val Glu Asn Lys
   1 5 10 15
<210> 37
   <211> 17
   <212> PRT
   <213> Artificial
<220>
   <223> SYNTHETIC PEPTI DE
<400> 37 Phe
<210> 38
   <211> 17
   <212> PRT
   <213> Artificial
<220>
   <223> SYNTHETIC PEPTI DE
<400> 38
   Ser Ser Thr Ser Glu Asn Asn Gly Asn Gly Asn Gly Asn Gly Gly Thr
   1 5 10 15
   Asp
<210> 39
   <211> 18
   <212> PRT
   <213> Artificial
<220>
   <223> SYNTHETIC PEPTI DE
<400> 39
   Gly Asn Asn Asp Glu Ser Asn Ile Ser Phe Lys Glu Lys Ser Glu Glu
   1 5 10 15
   Glu Glu
<210> 40
   <211> 18
   <212> PRT
   <213> Artificial
<220>
   <223> SYNTHETIC PEPTIDE
<400> 40
   Gly Asn Asn Asp Glu Ser Asn Ile Ser Phe Lys Glu Lys Ser Lys Lys
   1 5 10 15
   Lys Lys
<210> 41
   <211> 18
   <212> PRT
   <213> Artificial
<220>
   <223> SYNTHETIC PEPTI DE
<400> 41
   Gly Asn Asn Asp Glu Ser Asn Ile Ser Phe Lys Glu Lys Ser Pro Pro
   1 5 10 15
   Pro Pro
<210> 42
   <211> 18
   <212> PRT
   <213> Artificial
<220>
   <223> SYNTHETIC PEPTI DE
<400> 42
   Ser Ser Asn Lys Ser Thr Thr Gly Ser Gly Glu Thr Thr Thr Ala Ala
   1 5 10 15
   Gly Thr
<210> 43
   <211> 19
   <212> PRT
   <213> Artificial
<220>
   <223> SYNTHETIC PEPTI DE
<400> 43
   Cys Gly Asn Asn Asp Glu Ser Asn Ile Ser Phe Lys Glu Lys Ser Lys
   1 5 10 15
   Lys Lys Lys
<210> 44
   <211> 19
   <212> PRT
   <213> Artificial
<220>
   <223> SYNTHETIC PEPTI DE
<400> 44
   Gly Ser Pro Leu Ser Phe Glu Ser Ser Val Gln Leu Ile Val Ser Asp
   1 5 10 15
   Asn Ser Ser
<210> 45
   <211 > 19
   <212> PRT
   <213> Artificial
<220>
   <223> SYNTHETIC PEPTI DE
<400> 45
   Ser Asn Tyr Ala Lys Lys Val Val Lys Gln Lys Asn His Val Tyr Thr
   1 5 10 15
   Pro Val Tyr
<210> 46
   <211> 21
   <212> PRT
   <213> Artificial
<220>
   <223> SYNTHETIC PEPTI DE
<400> 46
   Ala Asp Val Ile Ala Lys Ile Val Glu Ile Val Lys Gly Leu Ile Asp
   1 5 10 15
   Gln Phe Thr Gln Lys
   20
<210> 47
   <211> 21
   <212> PRT
   <213> Artificial
<220>
   <223> SYNTHETIC PEPTI DE
<400> 47
   Gly Ala Ala Ser Ser Leu Thr Tyr Glu Ser Ser Val Gln Leu Val Val
   1 5 10 15
   Ser Asp Asn Ser Ser
   20
<210> 48
   <211 > 21
   <212> PRT
   <213> Artificial
<220>
   <223> SYNTHETIC PEPTI DE
<400> 48
   Gly Gly Glu Pro Ala Ala Gln Ala Pro Ala Glu Thr Pro Ala Ala Ala
   1 5 10 15
   Ala Glu Ala Ala Ser
   20
<210> 49
   <211> 20
   <212> PRT
   <213> Artificial
<220>
   <223> SYNTHETIC PEPTI DE
<400> 49
   Gly Gln Thr Asp Asn Asn Ser Ser Gln Ser Gln Gln Pro Gly Ser Gly
   1               5                   10                  15
   Thr Thr Asn Thr
               20
<210> 50
   <211> 22
   <212> PRT
   <213> Artificial
<220>
   <223> SYNTHETIC PEPTIDE
<400> 50
   Ser Gly Ala Leu Ala Ala Thr Ser Asp Asp Asp Val Lys Lys Ala Ala
   1               5                   10                  15
   Thr Val Ala Ile Val Ala
               20
<210> 51
   <211> 22
   <212> PRT
   <213> Artificial
<220>
   <223> SYNTHETIC PEPTI DE
<400> 51
   Ser Ile Val Ser Thr Ile Ile Glu Val Val Lys Thr Ile Val Asp Ile
   1               5                   10                  15
   Val Lys Lys Phe Lys Lys
               20
<210> 52
   <211> 22
   <212> PRT
   <213> Artificial
<220>
   <223> SYNTHETIC PEPTI DE
<400> 52
   Ser Ser Gly Gly Gly Gly Val Ala Ala Asp Ile Gly Ala Gly Leu Ala
   1 5 10 15
   Asp Ala Leu Thr Ala Pro
   20